# EUROPEAN PATENT APPLICATION

(11) **EP 4 715 062 A1**
(43) Date of publication of application: **25.03.2026**
(21) Application number: 24807282.9
(22) Date of filing: 17.05.2024
(51) Int. Cl.: C12Q 1/34, C12Q 1/04

(54) **BIOMARKER FOR DETERMINING STRESS LEVEL**

(30) Priority: 18.05.2023 JP 2023081936
(71) Applicant: National Institute of Advanced Industrial Science and Technology, Chiyoda-ku Tokyo 100-8921 (JP)
(72) Inventor: TATENO, Hiroaki, Tsukuba-shi, Ibaraki 305-8560 (JP); KEISHAM, Sunanda, Tsukuba-shi, Ibaraki 305-8560 (JP); OINAM, Lalhaba, Tsukuba-shi, Ibaraki 305-8560 (JP); YAMAGATA, Hirotaka, Ube-shi, Yamaguchi 755-8505 (JP); SEKI, Tomoe, Ube-shi, Yamaguchi 755-8505 (JP); KOBAYASHI, Ayumi, Ube-shi, Yamaguchi 755-8505 (JP)
(74) Representative: Latscha Schöllhorn Partner AG
(86) International application number: PCT/JP2024/018286
(87) International publication number: WO 2024/237332

(57) **Abstract**

The present invention addresses the problem of providing a biomarker for determining a stress level, a detection method for the biomarker, a data collection method including the detection method, and a kit for determining a stress level in order to determine a stress level received by a subject by means of a non-invasive and simple method. The above objective can be achieved by using, as a biomarker, sialidase activity from bacteria in a sample acquired from a subject. The sample is preferably stool and/or the subject is preferably human.

## Description

### TECHNICAL FIELD

The present invention relates to a biomarker for determining a stress level, a detection method for said biomarker, a data collection method including said detection method, and a kit for determining a stress level.

### BACKGROUND

In a study by the Japanese Ministry of Health, Labour and Welfare, the lifetime prevalence of depression was reported to be 3-16%, and the WHO has predicted, based on DALYs (Disability Adjusted Life Years (an indicator of disease burden)), that depression will become the disease that is the most damaging to health in 2030. The diagnosis and treatment of depression is an important issue for present-day society. The causes of depression can be complex and there is much that is unclear. However, excessive stress is known to be a factor.

Diagnoses of depression have conventionally been made by means of operational determination methods. Operational determination methods are diagnostic methods in which symptoms are matched to diagnostic criteria and determinations are made from the combination of symptoms. As the diagnostic criteria, those in the *International Classification of Diseases, version 10 (ICD-10),* by the WHO, the Diagnostic and Statistical Manual of Mental Disorders, fifth edition (DSM-5), by the American Psychiatric Association, etc. are used. However, there was a problem in that there was no testing method for objectively determining depression, depressive states, stress levels, etc.
As a numerical diagnostic method, there is optical topography (NIRS), by which objective testing is possible based on changes in the oxygenation state of brain hemoglobin in subjects suspected of having depression. However, it has not been adopted as a diagnostic criterion for depression as mentioned above, and it is currently only used as a supplementary method for discriminating between depression and bipolar disorder.
Additionally, an objective or quantitative evaluation method has not been established for stress, which is known to induce depression.

Markers for determining stress levels, methods for determining stress levels, markers for diagnosing depression, methods for diagnosing depression, etc. are being developed in order to enable objective evaluations of stress levels and objective diagnoses of depression. For example, as markers for diagnosing depression, those in which the degree of methylation of DNA sampled from blood is used as an indicator (Patent Document 1), those in which specific genes in blood are used as markers (Patent Document 2), those in which metabolites in blood are used as markers (Patent Document 3), etc. have been proposed.
Patent Document 1: JP 2019-193578 A
Patent Document 2: JP 2017-000063 A
Patent Document 3: WO 2017/082103 A

### SUMMARY OF INVENTION

However, none of these diagnostic markers or diagnostic methods have been clinically applied, and there are problems in that taking diagnostic samples is invasive and requires the intervention of an expert, specialized analysis technology is required for the diagnostic procedure, expensive and specialized equipment is required for the analysis, time is required until diagnostic results are obtained, etc.

A problem to be solved by the present invention is to provide a biomarker for determining a stress level, a stress level determination method, and a kit for determining a stress level in order to determine a stress level by means of a non-invasive and simple method.

As a result of diligent investigation, the present inventors discovered that sialidase activity from bacteria in a sample acquired from a subject is correlated with stress in the subject, and that the composition of bacterial species in a bacterial population having sialic acid in such a sample is correlated with stress in the subject, thereby completing the present invention.

The present invention has the aspects indicated below.
[1-1] A biomarker for determining a stress level received by a subject, wherein the biomarker is sialidase activity in a sample acquired from the subject.
[1-2] The biomarker according to [1-1], wherein the biomarker is sialidase activity from bacteria in the sample acquired from the subject.
[1-3] The biomarker according to [1-2], wherein the sample is stool.
[1-4] The biomarker according to any one of [1-1] to [1-3], wherein the subject is a human.
[1-5] The biomarker according to any one of [1-1] to [1-4], wherein the bacteria include bacteria in the class *Clostridia.*
[1-6] A biomarker detection method, wherein the method includes detecting the biomarker according to any one of [1-1] to [1-5] in the sample acquired from the subject.
[1-7] A data collection method for determining a stress level received by a subject, wherein the method includes the detection method according to [1-6].
[1-8] A kit for determining a stress level received by a subject, wherein the kit includes means for detecting the biomarker according to any one of [1-1] to [1-5].
[1-9] Use of sialidase activity in a sample acquired from a subject as a biomarker for determining a stress level received by the subject.
[1-10] Use of sialidase activity from bacteria in a sample acquired from a subject as a biomarker for determining a stress level received by the subject.
[1-11] Use according to [1-9] or [1-10], wherein the sample is stool.
[1-12] A method for determining a stress level received by a subject, wherein the method includes the data collection method according to [1-7].
[2-1] A biomarker for diagnosing depression in a subject, wherein the biomarker is sialidase activity in a sample acquired from the subject.
[2-2] The biomarker according to [2-1], wherein the biomarker is sialidase activity from bacteria in the sample acquired from the subject.
[2-3] The biomarker according to [2-2], wherein the sample is stool.
[2-4] The biomarker according to any one of [2-1] to [2-3], wherein the subject is a human.
[2-5] The biomarker according to any one of [2-1] to [2-4], wherein the bacteria include bacteria in the class *Clostridia.*
[2-6] A biomarker detection method, wherein the method includes detecting the biomarker according to any one of [2-1] to [2-5] in the sample acquired from the subject.
[2-7] A data collection method for diagnosing depression in a subject, wherein the method includes the detection method according to [2-6].
[2-8] A kit for diagnosing depression in a subject, wherein the kit includes means for detecting the biomarker according to any one of [2-1] to [2-5].
[2-9] Use of sialidase activity in a sample acquired from a subject as a biomarker for diagnosing depression in the subject.
[2-10] Use of sialidase activity from bacteria in a sample acquired from a subject as a biomarker for diagnosing depression in the subject.
[2-11] Use according to [2-9] or [2-10], wherein the sample is stool.
[2-12] A method for diagnosing depression in a subject, wherein the method includes the data collection method according to [2-7].
[2-13] A diagnostic method for depression in a subject, wherein the method includes the data collection method according to [2-7].
[3-1] A biomarker for evaluating efficacy of an antidepressant in a subject, wherein the biomarker is sialidase activity in a sample acquired from the subject.
[3-2] The biomarker according to [3-1], wherein the biomarker is sialidase activity from bacteria in the sample acquired from the subject.
[3-3] The biomarker according to [3-1] or [3-2], wherein the sample is stool.
[3-4] The biomarker according to any one of [3-1] to [3-3], wherein the subject is a human.
[3-5] The biomarker according to any one of [3-1] to [3-4], wherein the bacteria include bacteria in the class *Clostridia.*
[3-6] A biomarker detection method, wherein the method includes detecting the biomarker according to any one of [3-1] to [3-5] in the sample acquired from the subject.
[3-7] A data collection method for evaluating efficacy of an antidepressant in a subject, wherein the method includes the detection method according to [3-6].
[3-8] A kit for evaluating efficacy of an antidepressant in a subject, wherein the kit includes means for detecting the biomarker according to any one of [3-1] to [3-5].
[3-9] Use of sialidase activity in a sample acquired from a subject as a biomarker for evaluating efficacy of an antidepressant in the subject.
[3-10] Use of sialidase activity from bacteria in a sample acquired from a subject as a biomarker for evaluating efficacy of an antidepressant in the subject.
[3-11] Use according to [3-9] or [3-10], wherein the sample is stool.
[3-12] A method for evaluating efficacy of an antidepressant in a subject, wherein the method includes the data collection method according to [3-7].
[4-1] A biomarker for determining a stress level received by a subject, wherein the biomarker is a composition of a bacterial species in a bacterial population having sialic acid in a sample acquired from the subject.
[4-2] The biomarker according to [4-1], wherein the composition of the bacterial species in the bacterial population having sialic acid is an amount of bacteria in the class *Clostridia* contained in the bacterial population having sialic acid.
[4-3] The biomarker according to [4-1] or [4-2], wherein the sample is stool.
[4-4] The biomarker according to any one of [4-1] to [4-3], wherein the subject is a human.
[4-5] A biomarker detection method, wherein the method includes detecting the biomarker according to any one of [4-1] to [4-4] in the sample acquired from the subject.
[4-6] A data collection method for determining a stress level received by a subject, wherein the method includes the detection method according to [4-5].
[4-7] A kit for determining a stress level received by a subject, wherein the kit includes means for detecting the biomarker according to any one of [4-1] to [4-4].
[4-8] Use of a composition of a bacterial species in a bacterial population having sialic acid in a sample acquired from a subject as a biomarker for determining a stress level received by the subject.
[4-9] Use according to [4-8], wherein the composition of the bacterial species in the bacterial population having sialic acid is an amount of bacteria in the class *Clostridia* contained in the bacterial population having sialic acid.
[4-10] A method for determining a stress level received by a subject, wherein the method includes the data collection method according to [4-6].
[5-1] A biomarker for diagnosing depression in a subject, wherein the biomarker is a composition of a bacterial species in a bacterial population having sialic acid in a sample acquired from the subject.
[5-2] The biomarker according to [5-1], wherein the composition of the bacterial species in the bacterial population having sialic acid is an amount of bacteria in the class *Clostridia* contained in the bacterial population having sialic acid.
[5-3] The biomarker according to [5-1] or [5-2], wherein the sample is stool.
[5-4] The biomarker according to any one of [5-1] to [5-3], wherein the subject is a human.
[5-5] A biomarker detection method, wherein the method includes detecting the biomarker according to any one of [5-1] to [5-4] in the sample acquired from the subject.
[5-6] A data collection method for diagnosing depression in a subject, wherein the method includes the detection method according to [5-5].
[5-7] A kit for diagnosing depression in a subject, wherein the kit includes means for detecting the biomarker according to any one of [5-1] to [5-4].
[5-8] Use of a composition of a bacterial species in a bacterial population having sialic acid in a sample acquired from a subject as a biomarker for diagnosing depression in the subject.
[5-9] Use according to [5-8], wherein the composition of the bacterial species in the bacterial population having sialic acid is an amount of bacteria in the class *Clostridia* contained in the bacterial population having sialic acid.
[5-10] A method for diagnosing depression in a subject, wherein the method includes the data collection method according to [5-6].
[5-11] A diagnostic method for depression in a subject, wherein the method includes the data collection method according to [5-6].
[6-1] A biomarker for determining a stress level received by a subject, wherein the biomarker is a composition of a bacterial species in a bacterial population having sialic acid in a sample acquired from the subject.
[6-2] The biomarker according to [6-1], wherein the composition of the bacterial species is a composition of a bacterial species in the phylum *Firmicutes* (*p_Firmicutes*) in the bacterial population.
[7-1] A biomarker for diagnosing depression in a subject, wherein the biomarker is a composition of a bacterial species in a bacterial population in a sample acquired from the subject.
[7-2] The biomarker according to [7-1], wherein the composition of the bacterial species is a composition of a bacterial species in the phylum *Firmicutes* (*p_Firmicutes*) in the bacterial population.
[7-3] A diagnostic method for depression in a subject, wherein the method includes detecting the biomarker according to [7-1] or [7-2] in the sample acquired from the subject.

According to the present disclosure, a biomarker for determining a stress level, a detection method for said biomarker, and a kit for determining a stress level can be provided in order to determine a stress level received by a subject by means of a non-invasive and simple method.

According to the present disclosure, a biomarker for diagnosing depression, a detection method for said biomarker, and a kit for diagnosing depression can be provided in order to diagnose a depression patient by means of a non-invasive and simple method.

Additionally, according to the present disclosure, a biomarker for evaluating the efficacy of an antidepressant, a detection method for said biomarker, and a kit for evaluating the efficacy of an antidepressant can be provided in order to evaluate the efficacy of an antidepressant by means of a non-invasive and simple method.

### BRIEF DESCRIPTION OF DRAWINGS

FIG. 1 is a diagram indicating analysis results (principal coordinate analysis) of microbial compositions in stool samples from mice in a control group, a chronic ultra-mild stress (CUMS) group, and a CUMS + antidepressant-administered group in Example 2.
FIG. 2 is a graph indicating bacteria counts (relative amounts) of various bacterial species in stool samples from mice in the control group, the CUMS group, and the CUMS + antidepressant-administered group in Example 2.
FIG. 3 is a graph indicating DNA barcode amounts corresponding to the sialic acid-binding lectin *Sambucus nigra* Lectin (Elderberry) bark (SNA) in a control group, a CUMS group, and a CUMS + antidepressant-administered group in Example 3 (*: p < 0.05).
FIG. 4 is a graph indicating interaction times (seconds) with target mice in a sociability test for mice in a control group, a CUMS group, and a CUMS + antidepressant-administered group in Example 4 (* p < 0.05).
FIG. 5 is a graph indicating the correlation between the binding capacity of sialic acid-binding lectin SNA to bacterial cells in the stool samples from mice in the control group, the CUMS group, and the CUMS + antidepressant-administered group obtained in Example 3, and the interaction times (horizontal axis, seconds) with target mice in the sociability test for mice in the control group, the CUMS group, and the CUMS + antidepressant-administered group obtained in Example 4 (R = 0.67, p = 0.0026).

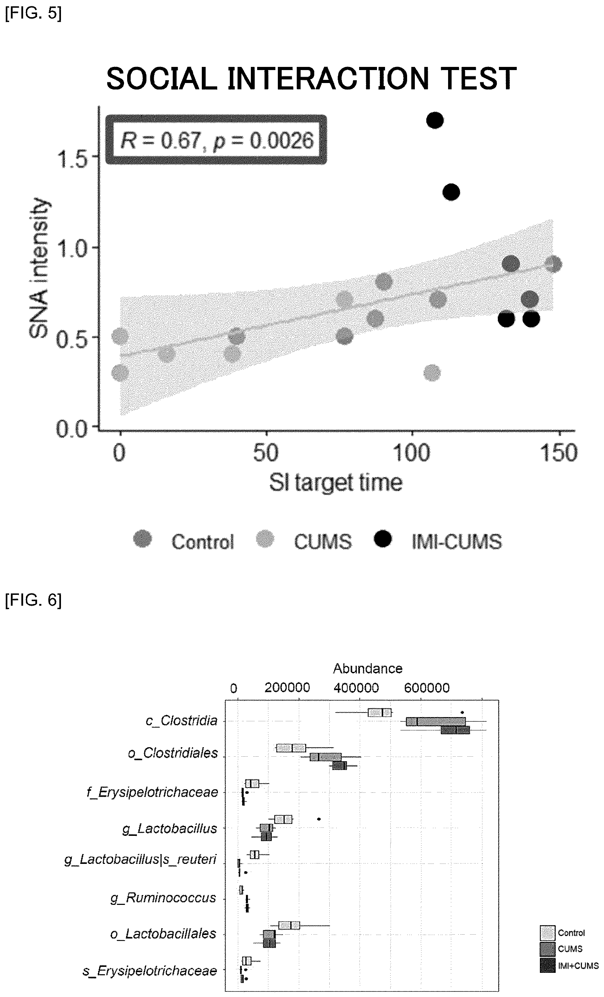

FIG. 6 is a graph indicating cell counts (relative amounts) of bacteria belonging to the sialylated class *Clostridia* (*c_Clostridia*), the order *Clostridiales* (*o_Clostridiales*), the family *Erysipelotrichaceae* (*f_Erysipelotrichaceae*), the genus *Lactobacillus* (*g_Lactobacillus*), *Lactobacillus reuteri* (*g_Lactobacillus s_reuteri*), the genus *Ruminococcus* (*g_Ruminococcus*), the order *Lactobacillales (o_Lactobacillales),* and the species *Erysipelotrichaceae (s_Erysipelotrichaceae)* for mice in a control group, a CUMS group, and a CUMS + antidepressant-administered group in Example 5.
FIG. 7, on the left side, is a graph indicating the correlation between the time (seconds) spent by mice in the center in an open-field test in Example 5, and the cell count of bacteria belonging to sialylated *Lactobacillus reuteri* (*g_Lactobacillus s_reuteri*) (R = 0.7, p = 0.0014), and on the right side, is a graph indicating the correlation between the time (seconds) spent by mice in the center in an open-field test in Example 5, and the cell count of bacteria belonging to the sialylated order *Clostridiales* (*o_Clostridiales*) (R = -0.55, p = 0.017).
FIG. 8 is a graph indicating the sialidase activity in stool samples from mice in a control group, a CUMS group, and a CUMS + antidepressant-administered group in Example 6.
FIG. 9 is a graph indicating the correlation between the immobility time (seconds, horizontal axis) in a forced swim test (FST) and the sialidase activity (vertical axis) in stool samples from mice in the control group, the CUMS group, and the CUMS + antidepressant-administered group in Example 6 (R = 0.85, p = 0.0034).
FIG. 10 is a graph indicating the correlation between the cell counts (relative amounts) of bacteria belonging to the class *Clostridia* (*c_Clostridia*) and the sialidase activity in stool samples from mice in the control group, the CUMS group, and the CUMS + antidepressant-administered group obtained in Example 6 (R = 0.8, p = 0.004).
FIG. 11 is a graph indicating the correlation between the cell counts (relative amounts) of bacteria belonging to the phylum *Firmicutes* (*p_Firmicutes*) and the sialidase activity in stool samples from mice in the control group, the CUMS group, and the CUMS + antidepressant-administered group obtained in Example 6 (R = 0.83, p = 0.011).
FIG. 12 is a graph indicating the sialidase activity in stool samples from a healthy person (Control) and a depression patient (Depressive) in Example 7.

### DESCRIPTION OF EMBODIMENTS

Hereinafter, an embodiment of the present disclosure will be explained in detail. The present disclosure is not limited to the embodiment below, and can be implemented by adding modifications, as appropriate, within a range not compromising the effects of the present invention.

In the cases in which there are no particular explanations in the embodiment and examples, a method described in a standard protocol collection such as J. Sambrook, E. F. Fritsch & T. Maniatis (Ed.), Molecular Cloning, A Laboratory Manual (3rd edition), Cold Spring Harbor Press, Cold Spring Harbor, New York (2001); or F. M. Ausubel, R. Brent, R. E. Kingston, D. D. Moore, J. G. Seidman, J. A. Smith, K. Struhl (Ed.), Current Protocols in Molecular Biology, John Wiley & Sons Ltd., etc., or a modification or an alteration thereof is used. Additionally, in the case in which a commercially available reagent kit or measurement device is used, in the absence of any particular explanation, the protocol attached thereto is used.

The respective configurations in the respective embodiments and combinations thereof, etc. are examples, and additions, omissions, substitutions, and other modifications of the configurations, as appropriate, are possible within a range not departing from the spirit of the present disclosure. The present disclosure is not limited by the embodiments and is only limited by the scope of the claims. Each aspect disclosed in the present specification can be combined with any other characteristics disclosed in the present specification.

In the case in which a specific explanation describing one embodiment also applies to another embodiment, there are cases in which that explanation will be omitted for the other embodiment. In the present disclosure, the expression "X-Y" regarding a numerical range means "at least X and at most Y".

### == First Embodiment (stress level determination marker, sialidase activity) ==

### [Biomarker]

The biomarker according to the first embodiment is sialidase activity in a sample acquired from a subject. The biomarker of the present embodiment can be used to determine the stress level received by a subject.

### (Sialidase activity)

Sialidase (also known as neuraminidase, acylneuraminyl hydrolase, or EC3.2.1.18) is an enzyme that is common in animals and in many microbes, and is a glycohydrolase that cleaves sialic acid α-ketosidically linked to terminals from glycoproteins, glycolipids, oligosaccharides, etc. In the present specification, sialidase is not limited by the sialic acid that is to be cleaved. Sialidase is known to be present in a wide range of organisms including humans (Lipnicanova et al., "Diversity of sialidases found in the human body - A review", International Journal of Biological Macromolecules, Vol. 148, 1 April 2020, pages 857-868).

For example, sialic acid is a collective name for the nine-carbon sugar, 2-keto-3-deoxynononic acid, having a carboxyl group at the carbon-1 position, or a collective name for N- and/or O-acyl derivatives having neuraminic acid as the core structure. In the present specification, sialic acid includes sialic acids in both the narrow sense and the broad sense, and includes N-acetylneuraminic acid (Neu5Ac), N-glycolylneuraminic acid (Neu5Gc), deaminoneuraminic acid (Kdn), etc. The sialic acid is also not limited by, for example, modifications thereof, and may be an O-acetylate, an O-sulfate, a lactone, or a lactam.

In the present specification, "sialidase activity" may refer to the presence or absence of sialidase activity, or to the level of sialidase activity. That is, in the present embodiment, the presence or absence of sialidase activity, or the level of sialidase activity, in a sample acquired from a subject can be used for determining a stress level received by a subject.

The sialidase activity is not limited, in terms of the origin thereof, as long as it is sialidase activity in a sample acquired from a subject. However, it is preferably from bacteria in the sample, more preferably from gut bacteria in the sample, and even more preferably from bacteria including bacteria in the class *Clostridia* (*c-Clostridia*). In this case, gut bacteria include any kind of bacteria that were living in the gut of the subject before the sample was acquired from the subject. The bacteria in the sample may be two or more species of bacteria.

In the case in which the bacteria include bacteria in the class *Clostridia* (*c_Clostridia*), they may include two or more species of bacteria belonging to the class *Clostridia (c_Clostridia),* or may include one or more bacteria belonging to the class *Clostridia (c_Clostridia)* and one or more bacteria belonging to one or more other classes.

### (Stress level)

Stress is held to be a psychosomatic functional change caused by various types of physical, environmental, or psychological stimuli or burdens, and is largely classified into acute stress and chronic stress, which is caused by sustained (chronic) stress burdens.

Regarding the stress level, "received a prescribed level of stress" and "received stress" (or "receiving a prescribed level of stress" or "receiving stress") refer to an individual or a subject having undergone at least some sort of psychosomatic functional change caused by some sort of stimulus or burden. The stress "level" may be the type or intensity of the stimulus or burden, or the level of the psychosomatic functional change caused by the stimulus or burden. It may be an absolute value or a relative value. It may be "high" or "low" in comparison with a reference value (reference range), a normal value (normal range), or a standard curve (standard range), or may be "absent" or "present", or may be a score assigned in accordance with a prescribed scoring method.

A chronic stress level can be evaluated in accordance with a method that would be well known to a person skilled in the art, and for example, can be scored based on a medical consultation or a questionnaire. For example, the stress level of a subject can be evaluated by being converted to points using the Brief Job Stress Questionnaire (57 items) or the Brief Job Stress Questionnaire, short version (23 items) in accordance with the *Stress Check Program Implementation Manual Based on the Industrial Safety and Health Law, revised version* (revised February 2021, Japanese Ministry of Health, Labour, and Welfare). With these examinations, points are assigned in categories such as "job stressors", "psychosomatic stress reactions", and "social support", and high-stress people can be identified based on suitably defined standards in each of these three categories or combinations thereof.

In the present specification, "received a prescribed stress level", "received stress" (or "receiving a prescribed stress level" or "receiving stress"), etc. may be a state in which a person is determined to be a high-stress person by, for example, a determination method based on prescribed standards that are suitably defined in the Brief Job Stress Questionnaire (57 items) or the Brief Job Stress Questionnaire, short version (23 items) mentioned above, and "not receiving a prescribed stress level", "not receiving stress", etc. may be a state in which a person is determined not to be a high-stress person by a similar method.

### (Sample)

The sample is not limited as long as it is acquired from a subject, but is preferably a sample from the gut of the subject. By using, as a biomarker, sialidase activity from bacteria included in a sample from the gut, the stress level received by a subject can be easily determined with high accuracy and/or precision. As samples from the gut, there are, for example, stool and intestinal mucosal tissue.

In this case, the stool is not limited as long as it includes biological samples from the gut of a subject, and broadly includes gut contents. For example, it includes both gut contents after they have been excreted outside the body of the subject (so-called "stool") and gut contents before excretion. From the aspect of being non-invasive and not requiring the intervention of a specialist when acquiring the sample, the sample from the gut is preferably stool excreted outside the body of the subject.

The region of the gut from which the gut contents come is not limited, and for example, may be the small intestine including the duodenum, the jejunum, and the ileum, the large intestine including the cecum, the appendix, the colon, and the rectum, etc. In this case, the large intestine includes, for example, the ascending colon, the transverse colon, the descending colon, the sigmoid colon, the upper rectum, the lower rectum, etc.

If the sample is stool, it should be sampled and stored in accordance with a method that would be well known to a person skilled in the art so as to suppress the intermixture of undesired matter that is not originally contained in stool and/or so that the sialidase activity in the stool does not decrease after excretion from the subject and until the sialidase activity is detected.

### (Subject)

The subject refers to an individual that is to be subjected to a stress level determination and is not limited as long as it is an organism requiring a stress level determination and in which biomarkers are constantly or temporarily present in the body. For example, it may be a mammal or may be another type of animal. The mammal may be a human or a non-human animal. The non-human animal species may, for example, be a monkey, a dog, a cat, a horse, a cow, a pig, a sheep, a goat, a rabbit, a guinea pig, a hamster, a mouse, and/or a rat, etc., and there are no limitations based on the purpose of use, such as livestock, pets, experimental animals, etc. However, it is preferably a mammal, and is more preferably a human.

In one embodiment, the biomarker for determining a stress level received by a subject according to the present embodiment is preferably sialidase activity from bacteria in a sample acquired from a human.

In one embodiment, the biomarker for determining a stress level received by a subject according to the present embodiment is more preferably sialidase activity from bacteria in stool acquired from a human.

In one embodiment, the biomarker for determining a stress level received by a subject according to the present embodiment is even more preferably sialidase activity from bacteria including bacteria in the class *Clostridia (c_Clostridia)* in stool acquired from a human.

### [Detection method]

The detection method in one embodiment is a biomarker detection method for detecting any one of the biomarkers described in the above [Biomarker] section in the present embodiment in a sample acquired from a subject.

Due to the detection method described below, any one of the biomarkers described in the above [Biomarker] section in the present embodiment can be detected in a sample acquired from a subject.

Regarding the examples and the preferred examples of the "sialidase activity", the "sample", and the "subject", they are as described in the above [Biomarker] section in the present embodiment.

The acquisition of the sample from the subject can be selected, as appropriate, in accordance with methods that would be well known to a person skilled in the art. For example, in the case in which the sample is stool excreted from a subject, the stool should at least partially be acquired by using a sterilized instrument, etc. For example, in the case in which the sample is gut contents before being excreted from a subject, it can be acquired by pressing a container to the anus of the subject, or by inserting an instrument, such as a sterilized cotton swab or a sterilized dropper, into the anus of the subject and causing the sample to be deposited on the instrument. For example, in the case in which the sample is intestinal mucosal tissue from a subject, it can be acquired by inserting a sterilized instrument into the anus of the subject in a manner similar to the gut contents, and scraping intestinal mucosal tissue with the instrument.

Regarding pretreatments preceding biomarker detection, a pretreatment suitable for detection may be performed on the sample acquired from the subject, or the detection may be performed on the untreated sample. The detection pretreatment procedure can be selected, as appropriate, in accordance with a method that would be well known to a person skilled in the art depending on the type of sample, the amount or ratio of the biomarker contained in the sample, etc. For example, in the case in which the sample is stool or gut contents, the stool or gut contents, in solid form, excreted from the subject may be used for biomarker detection while still solid, or may be dissolved in a solution, etc. and made uniform, then a portion thereof may be used for biomarker detection in the liquid state. For example, in the case in which the sample is intestinal mucosal tissue, a mucosal tissue piece acquired from a subject may be used for biomarker detection while still in solid form, or may be used for biomarker detection after preparing a microscopic piece by means of homogenization or ultrasonic treatment, etc. Furthermore, in the case in which the sialidase activity is from bacteria, a desired bacterial population may be isolated from the sample and used for biomarker detection in accordance with a well-known method.

The biomarker detection is not limited as long as sialidase activity is detected, and may include, for example, measuring the presence or absence of sialidase activity, or measuring the level of sialidase activity. Considering the accuracy and/or precision of the diagnosis, the biomarker detection method according to the present embodiment preferably includes not only measurement of the presence or absence of sialidase activity, but also includes measurement of the level of sialidase activity.

In the present specification, the biomarker level (level of sialidase activity) is also referred to as the biomarker measurement value, and may be any one of the presence or absence of a biomarker in the sample, an absolute value, and a relative value.

In the present embodiment, the level of sialidase activity may be an absolute value or a relative value of enzyme activity.

In this case, the level of sialidase activity may be represented, for example, by "international units" (a single unit representing the amount of the enzyme that can convert 1 µmol of a substrate per minute at a temperature of 30 °C in 1 L of a sample under optimal conditions) which are generally used as units of enzyme activity.

The detection of sialidase activity can be performed, as appropriate, in accordance with a method that would be well known to a person skilled in the art.

For example, by using a substance that is a sialidase reaction substrate, products obtained by an enzyme reaction due to sialidase in a sample can be produced and the amount of decrease in the reaction substrate and/or the amount of increase in the product can be measured to measure the presence or absence of sialidase activity in the sample or to measure the level of sialidase activity in the sample.

The detection of sialidase activity can be performed by using a commercially available sialidase activity measurement kit that has been designed by making use of a mechanism as described above, so as to measure the presence or absence of sialidase activity or to measure the level of sialidase activity in accordance with the attached instructions. An example of such a commercially available kit is a neuraminidase activity measurement kit (commercially available from Sigma-Aldrich, etc.).

Instead of detecting sialidase activity itself, the sialidase activity may be detected by detecting a parameter correlated with the absolute value or the relative value of sialidase activity.

The parameter correlated with sialidase activity may, for example, be the protein mass of sialidase, which is thought to be correlated with the level of sialidase activity.

That is, in one embodiment, the biomarker detection method includes measuring the protein mass of sialidase in a sample acquired from a subject.

The protein mass of sialidase can be measured in accordance with a method that would be well known to a person skilled in the art. For example, the measurement may be performed by a well-known method using an antibody or probe that specifically binds to sialidase, such as an enzyme-linked immunosorbent assay (ELISA) method, whether a directly competitive method, an indirectly competitive method, or a sandwich method, a radioimmunoassay (RIA) method, a flow cytometry method, immunochromatography, etc.

Furthermore, the parameter correlated with sialidase activity may, for example, be the level of glycans having sialic acid. The level of glycans refers to an absolute or relative amount of sialylated glycans contained in a sample acquired from a subject. When the sialidase activity in a sample increases, the cleaving of sialic acid due to sialidase becomes more active. Accordingly, the level of glycans having sialic acid contained in a sample becomes lower in correlation with an increase in the level of sialidase activity. In this case, a glycan having sialic acid is, for example, a cell surface glycan that has been sialylated in bacteria contained in a sample. Sialylation (sialic acid modification) refers, in an N-linked glycan, to the linking of sialic acid to the 6-position or to the 3-position of non-reducing terminal galactose, and in an O-linked glycan, to the linking of sialic acid to the 6-position, to Core 1 (T), or to Core 1 in N-acetylgalactosamine.

That is, in one embodiment, the biomarker detection method includes measuring the level of glycans having sialic acid in a sample acquired from a subject.

The level of glycans having sialic acid can be measured in accordance with a method that would be well known to a person skilled in the art, or may be quantified by using, for example, a specific lectin that binds to sialylated glycans. As a more specific example, it may be measured by using the glycan sequencing (Glycan-seq) method developed by Oinam et al. ("Glycan Profiling of the gut microbiota by Glycan-seq", ISME Communications, 2022 Jan 5, 2, Article number: 1, doi.org/10.1038/s43705-021-00084-2).

According to the above-mentioned detection method, the acquisition of the sample is non-invasive and does not require the intervention of a specialist, and compared with methods requiring blood sampling or a biopsy, the above-mentioned detection method enables more convenient biomarker detection and result acquisition. For example, the subject him/herself can perform the steps from acquiring the sample to detecting the biomarker, and can also acquire test results.

Additionally, by conveniently measuring and detecting biomarkers, objective and/or quantitative test results can be acquired.

### [Determination method]

The data collection method according to one embodiment is a data collection method for determining a stress level received by a subject, including a detection method described in the above [Detection method] section in the present embodiment.

In one embodiment, the stress level received by a subject can be determined based on data collected by said data collection method. That is, the method for determining a stress level received by a subject according to one embodiment includes said data collection method.

The detection of sialidase activity, which is a biomarker, can be performed by means of any one of the detection methods described in the above [Detection method] section in the present embodiment.

In one embodiment, the data collection method for determining a stress level received by a subject may be a method that includes, before implementing any one of the detection methods described in the above [Detection method] section in the present embodiment on a sample acquired from a subject, implementing a biomarker detection method according to the above [Detection method] section in the present embodiment on a sample(s) acquired from one or more individuals that have received a prescribed level of stress, and acquiring a biomarker reference value based on the biomarker measurement value(s) in the individual(s) that has (have) received the prescribed level of stress.

In another embodiment, the data collection method for determining a stress level received by a subject may be a method that includes, before implementing any one of the detection methods described in the above [Detection method] section in the present embodiment on a sample acquired from a subject, implementing a biomarker detection method according to the above [Detection method] section in the present embodiment on a sample(s) acquired from one or more individuals that have received a prescribed level of stress and a sample(s) from one or more individuals that have not received the prescribed level of stress, and acquiring a biomarker reference value for an individual(s) that has (have) received the prescribed level of stress and a biomarker reference value (normal value) for an individual(s) that has (have) not received the prescribed level of stress.

According to these methods, in the case in which a biomarker measurement value in a sample acquired from a subject is the same as a biomarker reference value for an individual(s) that has (have) received a prescribed level of stress, based on the data, the subject may be determined to have received or to be receiving the prescribed level of stress, or may be determined to have a high probability of having received or receiving the prescribed level of stress. Alternatively, in the case in which a biomarker measurement value in a sample acquired from a subject is different from a biomarker reference value for an individual(s) that has (have) received a prescribed level of stress, based on the data, the subject may be determined to not have received the prescribed level of stress, or may be determined to have a high probability of not having received the prescribed level of stress.

Additionally, in the case in which a biomarker measurement value in a sample acquired from a subject is different from a normal value, based on the data, the subject may be determined to have received or to be receiving a prescribed level of stress, or to have a high probability of having received or receiving a prescribed level of stress. Alternatively, in the case in which a biomarker measurement value in a sample acquired from a subject is the same as a normal value, based on the data, the subject may be determined to not have received a prescribed level of stress, or to have a high probability of not having received a prescribed level of stress.

In one embodiment, in the case in which a biomarker measurement value in a sample acquired from a subject is elevated in comparison with a normal value, based on the data, the subject may be determined to have received or to be receiving a prescribed level of stress, or to have a high probability of having received or receiving a prescribed level of stress. This is because the sialidase activity in a sample acquired from an individual that has received stress is higher or tends to be higher than the sialidase activity in a sample acquired from an individual that has not received stress.

In another embodiment, the data collection method for determining a stress level received by a subject may be a method that includes, before implementing any one of the detection methods described in the above [Detection method] section in the present embodiment on a sample acquired from a subject, implementing a biomarker detection method according to the above [Detection method] section in the present embodiment on samples acquired from multiple individuals that have received respectively different levels of stress or samples acquired from multiple groups of individuals that have received respectively different levels of stress, and setting a standard curve indicating the relationship between stress levels and biomarker measurement values based on the biomarker measurement values in the individuals or the groups of individuals that have received different levels of stress.

According to this method, in the case in which a biomarker measurement value in a sample acquired from a subject is the same as any point lying inside the standard curve, based on the data, the subject may be determined to have received or to be receiving stress at a level corresponding to said point, or may be determined to have a high probability of having received or receiving the prescribed level of stress. In the case in which a biomarker measurement value in a sample acquired from a subject is different from any of the points lying inside the standard curve, based on the data, the subject may be determined to not have received stress at a level corresponding to any of the points lying inside the standard curve, or may be determined to have a high probability of not having received stress at a level corresponding to any of the points lying inside the standard curve.

In another embodiment, the data collection method for determining a stress level received by a subject may be a method that includes implementing any one of the detection methods described in the above [Detection method] section in the present embodiment on multiple samples acquired over time from the same subject.

According to this method, in the case in which the biomarker measurement values in the samples acquired from the subject exhibit a change at a certain time compared to before that time, based on the data, the subject may be determined to have received or to be receiving a prescribed level of stress, or to have a high probability of having received or receiving a prescribed level of stress. For example, through periodically implemented health checkups, etc., this method can be used to determine and to monitor stress levels received or being received by a subject.

In one embodiment, in the case in which the biomarker measurement values in the samples acquired from the subject increase at a certain time compared to before that time, based on the data, the subject may be determined to have received or to be receiving a prescribed level of stress, or to have a high probability of having received or receiving a prescribed level of stress. This is because the sialidase activity in a sample acquired from an individual that has received stress is higher or tends to be higher than the sialidase activity in a sample acquired from an individual that has not received stress.

In one embodiment, the stress level received by a subject can be determined by any one of the above-mentioned determinations.

In this case, the reference value, the normal value, or the standard curve may be set based on the mean value or the median value of biomarker measurement values in samples acquired from individuals in the same group or acquired over time from the same individual, or may be a reference range, a normal range, or a standard range that has been suitably set based on biomarker measurement values.

Additionally, the reference value, the normal value, or the standard curve may be decided, as appropriate, by a person skilled in the art to be a value by which the stress level received by a subject can be determined with a desired accuracy and/or precision based on biomarker measurement values in samples acquired from individuals in the same group or acquired over time from the same individual.

Additionally, the biomarker measurement value in a sample acquired from a subject being the same as a reference value, a normal value, or any point lying inside a standard curve may, for example, mean that there is no significant difference from the reference value or the normal value by a suitable statistical process. The biomarker measurement value in a sample acquired from a subject being different from a reference value, a normal value, or a point lying inside a standard curve may, for example, mean that there is a significant difference from the reference value, the normal value, or the point lying inside the standard curve by a suitable statistical process. The biomarker measurement value in a sample acquired from a subject being elevated in comparison with a reference value, for example, means that the biomarker measurement value in the sample acquired from the subject is significantly elevated in comparison with the reference value by a suitable statistical process.

The statistical processing method can be selected, as appropriate, in accordance with a method that would be well known to a person skilled in the art.

In one embodiment, the reference value may be a value that is 150%, 200%, 250%, 300%, 350%, 400%, 450%, or 500% or more with respect to the normal value, or may be decided, as appropriate, by a person skilled in the art to be a value by which the stress level received by a subject can be determined with a desired accuracy and/or precision.

In one embodiment, the data collection method explained above or the method for determining a stress level received by a subject based on said data collection method may be implemented in combination with another data collection method for determining a stress level received by a subject, or another method for determining a stress level received by a subject.

According to the above-mentioned determination method, the acquisition of the sample is non-invasive and does not require the intervention of a specialist, and compared with methods requiring blood sampling or a biopsy, the above-mentioned detection method enables more convenient biomarker detection and result acquisition. For example, the subject him/herself can perform the steps from acquiring the sample to detecting the biomarker, and can also acquire determination results.

Additionally, by conveniently measuring and detecting biomarkers, objective and/or quantitative determination results can be acquired.

### [Kit]

The kit in one embodiment is a kit for determining a stress level received by a subject, the kit including means for detecting any one of the biomarkers described in the above [Biomarker] section in the present embodiment.

In one embodiment, said kit is a kit for implementing a detection method described in the above [Detection method] section in the present embodiment.

In one embodiment, said kit may include, as the means for detecting the biomarker, for example, a sialidase reaction substrate, a reagent for detecting the amount of decrease of the reaction substrate/the amount of increase of a product, a positive control reagent indicating that the detection step is functioning, and/or a storage liquid for storing the sample acquired from the subject with sialidase until the detection method is implemented, and/or instructions for using the kit. In this case, the reaction substrate may be provided in a state of being attached to a container, a chip, etc.

In one embodiment, said kit may include a detection device necessary for detection, or may be provided together with said detection device.

In one embodiment, at least a portion of said kit may be provided in a state of being installed on a portion of a toilet bowl. By being installed on a toilet bowl, the sialidase activity in stool excreted from a subject can be easily used for health management.

According to the above-mentioned kit, the acquisition of the sample is non-invasive and does not require the intervention of a specialist, and compared with methods requiring blood sampling or a biopsy, the above-mentioned kit enables more convenient biomarker detection and result acquisition. For example, the subject him/herself can perform the steps from acquiring the sample to detecting the biomarker, and can also acquire determination results.

Additionally, by conveniently measuring and detecting biomarkers, objective and/or quantitative determination results can be acquired.

### == Second Embodiment (depression diagnosis marker, sialidase activity) ==

### [Biomarker]

The biomarker according to the second embodiment is sialidase activity in a sample acquired from a subject. The biomarker of the present embodiment can be used to diagnose depression in a subject.

Depression is known to be caused by stress. Thus, sialidase activity in a sample acquired from a subject, which is the biomarker according to the first embodiment, can be used to diagnose depression in a subject.

### (Depression)

In the present specification, "depression" broadly covers not only diseases satisfying the major depressive disorder diagnostic criteria under the diagnostic criteria in the International Classification of Diseases, version 10 (ICD-10) by the WHO or the Diagnostic and Statistical Manual of Mental Disorder, fifth edition (DSM-5) by the American Psychiatric Association, but also various types of diseases and symptoms associated with mood disorders, and adjustment disorders, as long as a depressive state is at least temporarily exhibited. There is no limitation based on the time period over which the disease occurs or symptoms are exhibited, the severity thereof, and/or the causes thereof (for example, being caused by genetic and external stress, as well as diseases other than mental diseases, such as infectious diseases, neoplastic diseases, neurological diseases, etc.). As the various types of diseases and symptoms associated with mood disorders in which a depressive state is at least temporarily exhibited mentioned above, there are, for example, depressive mood disorders, bipolar mood disorders, etc. As depressive mood disorders other than major depression, there are depression not otherwise specified (NOS), dysthymia (persistent depressive disorder), etc. As bipolar mood disorders, there are bipolar I disorder, bipolar II disorder, bipolar disorder not otherwise specified (NOS), etc. From the aspect of diagnosing depression in a subject with higher accuracy and/or precision, the disease to be diagnosed is preferably a depressive mood disorder (including major depression, depression NOS, and dysthymia), or an adjustment disorder, and is more preferably major depression, depression NOS, or an adjustment disorder. The subject may exhibit any of the symptoms that are observed in anxiety disorders, sleep disorders, eating disorders, etc., as long as they are mood disorders in which a depressive state is at least temporarily exhibited. The subject may suffer from a disease other than a mental disorder, such as an infectious disease, a neoplastic disease, a neurological disease, etc.

Additionally, language such as "depressive disease", etc. in the present specification covers not only depression, but also situations in which there is a high probability of depression. Additionally, in the present specification, language such as "diagnosis of depression", "diagnosing depression", and "depression diagnosis" includes not only determining whether or not the subject is suffering from depression, but also whether or not there is a high probability that the subject is suffering from depression.

Language such as "health", "healthy individual", etc. in the context of the "depression" in the present specification refers to an individual not being diagnosed with depression or having a low probability of suffering from depression. Cases in which an individual is suffering from a disease other than depression are also included.

### (Sialidase activity)

Examples and preferred examples of "sialidase activity" are as described in the "Sialidase activity" portion of the [Biomarker] section in the first embodiment.

### (Sample)

Examples and preferred examples of the "sample" are as described in the "Sample" portion of the [Biomarker] section in the first embodiment.

### (Subject)

The "subject" refers to an individual that is to be diagnosed for depression, and aside from the fact that the subject is in need of a diagnosis for depression, the examples and preferred examples thereof are as described in the "Subject" portion of the [Biomarker] section in the first embodiment.

### [Detection method]

The detection method in one embodiment is a biomarker detection method for detecting any one of the biomarkers described in the above [Biomarker] section in the present embodiment in a sample acquired from a subject.

In this case, regarding the examples and the preferred examples of the "sialidase activity", the "sample", and the "subject", they are as described in the above [Biomarker] section in the present embodiment.

In the present embodiment, the acquisition of a sample from a subject, the pretreatment preceding biomarker detection, and the biomarker detection are as described in the [Detection method] section in the first embodiment.

According to the above-mentioned detection method, the acquisition of the sample is non-invasive and does not require the intervention of a specialist, and compared with methods requiring blood sampling or a biopsy, the above-mentioned detection method enables more convenient biomarker detection and result acquisition. For example, the subject him/herself can perform the steps from acquiring the sample to detecting the biomarker, and can also acquire test results.

Additionally, by conveniently measuring and detecting biomarkers, objective and/or quantitative test results can be acquired.

### [Diagnostic method]

The data collection method according to one embodiment is a data collection method for diagnosing depression in a subject, including a detection method described in the above [Detection method] section in the present embodiment.

The detection of sialidase activity, which is a biomarker, can be performed by means of any one of the detection methods described in the above [Detection method] section in the present embodiment.

In one embodiment, the data collection method for diagnosing depression in a subject may be a method that includes, before implementing any one of the detection methods described in the above [Detection method] section in the present embodiment on a sample acquired from a subject, implementing a biomarker detection method according to the above [Detection method] section in the present embodiment on a sample(s) acquired from one or more depression patients, and acquiring a biomarker reference value based on the biomarker measurement value(s) in the depression patient(s).

In another embodiment, the data collection method for diagnosing depression in a subject may be a method that includes, before implementing any one of the detection methods described in the above [Detection method] section in the present embodiment on a sample acquired from a subject, implementing a biomarker detection method according to the above [Detection method] section in the present embodiment on a sample(s) acquired from one or more depression patients and a sample(s) acquired from one or more healthy individuals, and acquiring a biomarker reference value for a depression patient(s) and a biomarker reference value (normal value) for a healthy individual(s).

According to these methods, in the case in which a biomarker measurement value in a sample acquired from a subject is the same as a biomarker reference value for a depression patient(s), based on the data, the subject may be determined to be suffering from depression, or may be determined to have a high probability of suffering from depression. Alternatively, in the case in which a biomarker measurement value in a sample acquired from a subject is different from a biomarker reference value for a depression patient(s), based on the data, the subject may be determined to not be suffering from depression, or may be determined to have a low probability of suffering from depression.

Additionally, in the case in which a biomarker measurement value in a sample acquired from a subject is different from a normal value, based on the data, the subject may be determined to be suffering from depression, or may be determined to have a high probability of suffering from depression. Alternatively, in the case in which a biomarker measurement value in a sample acquired from a subject is the same as a normal value, based on the data, the subject may be determined to not be suffering from depression, or may be determined to have a low probability of suffering from depression.

In one embodiment, in the case in which a biomarker measurement value in a sample acquired from a subject is elevated in comparison with a normal value, based on the data, the subject may be determined to be suffering from depression, or may be determined to have a high probability of suffering from depression. This is because the sialidase activity in a sample acquired from a depression patient is higher or tends to be higher than the sialidase activity in a sample acquired from a healthy individual.

In another embodiment, the data collection method for diagnosing depression in a subject may be a method that includes implementing any one of the detection methods described in the above [Detection method] section in the present embodiment on multiple samples acquired over time from the same subject.

According to this method, in the case in which the biomarker measurement values in the samples acquired from the subject exhibit a change at a certain time compared to before that time, based on the data, the subject may be determined to be suffering from depression or to have a high probability of suffering from depression. For example, through periodically implemented health checkups, etc., this method can be used to monitor depression in a subject.

In one embodiment, in the case in which the biomarker measurement values in the samples acquired from the subject increase at a certain time compared to before that time, based on the data, the subject may be determined to be suffering from depression or to have a high probability of suffering from depression. This is because the sialidase activity in a sample acquired from a depression patient is higher or tends to be higher than the sialidase activity in a sample acquired from a healthy individual.

In one embodiment, depression can be diagnosed in a subject by any one of the above-mentioned determinations.

In this case, the reference value or the normal value may be set based on the mean value or the median value of biomarker measurement values in samples acquired from individuals in the same group or acquired over time from the same individual, or may be a reference range or a normal range that has been suitably set based on biomarker measurement values.

Additionally, the reference value or the normal value may be decided, as appropriate, by a person skilled in the art to be a value by which a subject can be determined to be suffering from depression with a desired accuracy and/or precision based on biomarker measurement values in samples acquired from individuals in the same group or acquired over time from the same individual.

Additionally, the biomarker measurement value in a sample acquired from a subject being the same as a reference value or a normal value may, for example, mean that there is no significant difference from the reference value or the normal value by a suitable statistical process. The biomarker measurement value in a sample acquired from a subject being different from a reference value or a normal value may, for example, mean that there is a significant difference from the reference value or the normal value by a suitable statistical process. The biomarker measurement value in a sample acquired from a subject being elevated in comparison with a reference value may, for example, mean that the biomarker measurement value in the sample acquired from the subject is significantly elevated in comparison with the reference value by a suitable statistical process.

The statistical processing method can be selected, as appropriate, in accordance with a method that would be well known to a person skilled in the art.

In one embodiment, the reference value may be a value that is 150%, 200%, 250%, 300%, 350%, 400%, 450%, or 500% or more with respect to the normal value, or may be decided, as appropriate, by a person skilled in the art to be a value by which a subject can be determined to be suffering from depression with a desired accuracy and/or precision.

In one embodiment, the data collection method explained above or the method for diagnosing depression based on said data collection method may be implemented in combination with another data collection method for diagnosing depression, or another method for diagnosing depression.

According to the above-mentioned evaluation method, the acquisition of the sample is non-invasive and does not require the intervention of a specialist, and compared with methods requiring blood sampling or a biopsy, the above-mentioned evaluation method enables more convenient biomarker detection and result acquisition. For example, the subject him/herself can perform the steps from acquiring the sample to detecting the biomarker, and can also acquire diagnosis results.

Additionally, by conveniently measuring and detecting biomarkers, objective and/or quantitative diagnosis results can be acquired.

### == Third Embodiment (antidepressant efficacy evaluation marker, sialidase activity) ==

### [Biomarker]

The biomarker according to the third embodiment is sialidase activity in a sample acquired from a subject. The biomarker of the present embodiment can be used to evaluate the efficacy of an antidepressant in a subject.

As explained in the second embodiment, sialidase activity in a sample acquired from a subject can be used as a biomarker for diagnosing depression in the subject. Therefore, by using sialidase activity as a biomarker, it can be determined whether or not depression in a subject improved after administering an antidepressant, i.e., the efficacy of the antidepressant in the subject can be evaluated.

### (Sialidase activity)

Examples and preferred examples of "sialidase activity" are as described in the "Sialidase activity" portion of the [Biomarker] section in the first embodiment.

### (Antidepressant)

In the present specification, the "antidepressant" includes approved and unapproved pharmaceuticals and therapeutic methods used to treat depression in Japan and in other countries. For example, they include selective serotonin reuptake inhibitors (SSRIs), serotonin-noradrenaline reuptake inhibitors (SNRIs), electroconvulsive therapies, and/or transcranial magnetic stimulation, etc. Selective serotonin reuptake inhibitors include, for example, escitalopram, sertraline, paroxetine, etc., and serotonin-noradrenaline reuptake inhibitors include, for example, imipramine, venlafaxine, etc.

### (Sample)

Examples and preferred examples of the "sample" are as described in the "Sample" portion of the [Biomarker] section in the first embodiment.

### (Subject)

The "subject" refers to an individual that is to be evaluated for efficacy of an antidepressant, and aside from the fact that the subject is in need of an evaluation for efficacy of an antidepressant, the examples and preferred examples thereof are as described in the "Subject" portion of the [Biomarker] section in the first embodiment. Since the biomarker according to the present embodiment is a biomarker for evaluating the efficacy of an antidepressant, the subject is preferably a depression patient or an individual suspected to be suffering from depression. The individual suspected to be suffering from depression may be an individual that has received or is receiving a prescribed level of stress.

### [Detection method]

The detection method in one embodiment is a biomarker detection method for detecting any one of the biomarkers described in the above [Biomarker] section in the present embodiment in a sample acquired from a subject.

In this case, regarding the examples and the preferred examples of the "sialidase activity", the "sample", and the "subject", they are as described in the above [Biomarker] section in the present embodiment.

In the present embodiment, the acquisition of a sample from a subject, the pretreatment preceding biomarker detection, and the biomarker detection are as described in the [Detection method] section in the first embodiment.

According to the above-mentioned detection method, the acquisition of the sample is non-invasive and does not require the intervention of a specialist, and compared with methods requiring blood sampling or a biopsy, the above-mentioned detection method enables more convenient biomarker detection and result acquisition. For example, the subject him/herself can perform the steps from acquiring the sample to detecting the biomarker, and can also acquire test results.

Additionally, by conveniently measuring and detecting biomarkers, objective and/or quantitative test results can be acquired.

### [Evaluation method]

The data collection method according to one embodiment is a data collection method for evaluating the efficacy of an antidepressant in a subject including the detection method described in the above [Detection method] section in the present embodiment.

In one embodiment, the efficacy of an antidepressant in a subject can be evaluated based on data collected by said data collection method. That is, the method for evaluating the efficacy of an antidepressant in a subject according to one embodiment includes said data collection method.

The detection of sialidase activity, which is a biomarker, can be performed by means of any one of the detection methods described in the above [Detection method] section in the present embodiment.

In one embodiment, the data collection method for evaluating the efficacy of an antidepressant in a subject may be a method that includes, before implementing the above [Detection method] in the present embodiment on a sample acquired from a subject, implementing a biomarker detection method according to the above [Detection method] section in the present embodiment on a sample(s) acquired from one or more healthy individuals or depression patients (including former depression patients that have recovered due to treatment) in whom effects of an antidepressant have been obtained, and acquiring a biomarker reference value for a depression patient(s) in whom the effects of the antidepressant have been obtained.

In another embodiment, the data collection method for evaluating the efficacy of an antidepressant in a subject may be a method that includes, before implementing any one of the detection methods described in the above [Detection method] section in the present embodiment on a sample acquired from a subject, implementing a biomarker detection method according to the above [Detection method] section in the present embodiment on a sample(s) acquired from one or more depression patients in whom effects of an antidepressant were not obtained or depression patients that have not received treatment by said antidepressant, and acquiring a biomarker reference value for a depression patient(s) in whom the effects of the antidepressant were not obtained or a depression patient(s) that has (have) not received treatment by said antidepressant.

In another embodiment, the data collection method for evaluating the efficacy of an antidepressant in a subject may be a method that includes, before implementing any one of the detection methods described in the above [Detection method] section in the present embodiment on a sample acquired from a subject, implementing a biomarker detection method according to the above [Detection method] section in the present embodiment on a sample(s) acquired from one or more healthy individuals or depression patients (including former depression patients that have recovered due to treatment) in whom effects of an antidepressant have been obtained, and one or more depression patients in whom effects of an antidepressant were not obtained or depression patients that have not received treatment by said antidepressant, and acquiring a biomarker reference value (first reference value) for a healthy individual(s) or a depression patient(s) in whom the effects of the antidepressant have been obtained, and a biomarker reference value (second reference value) for a depression patient(s) in whom the effects of the antidepressant were not obtained or a depression patient(s) that has (have) not received treatment by said antidepressant.

According to these methods, in the case in which a biomarker measurement value in a sample acquired from a subject is the same as a biomarker reference value for a healthy individual(s) or a depression patient(s) in whom effects of an antidepressant have been obtained, based on the data, the subject may be determined to have obtained the effects of the antidepressant, or may be determined to have a high probability of having obtained the effects of the antidepressant. Alternatively, in the case in which a biomarker measurement value in a sample acquired from a subject is different from a biomarker reference value for a healthy individual(s) or a depression patient(s) in whom effects of an antidepressant have been obtained, based on the data, the subject may be determined to have not obtained the effects of the antidepressant, or may be determined to have a high probability of not having obtained the effects of the antidepressant.

Additionally, in the case in which a biomarker measurement value in a sample acquired from a subject is different from a biomarker reference value for a depression patient(s) in whom effects of an antidepressant were not obtained or a depression patient(s) that has (have) not received treatment by said antidepressant, based on the data, the subject may be determined to have obtained the effects of the antidepressant, or may be determined to have a high probability of having obtained the effects of the antidepressant. Alternatively, in the case in which a biomarker measurement value in a sample acquired from a subject is the same as a biomarker reference value for a depression patient(s) in whom effects of an antidepressant were not obtained or a depression patient(s) that has (have) not received treatment by said antidepressant, based on the data, the subject may be determined to have not obtained the effects of the antidepressant, or may be determined to have a high probability of not having obtained the effects of the antidepressant.

In one embodiment, in the case in which a biomarker measurement value in a sample acquired from a subject is lower than a biomarker reference value for a depression patient(s) in whom effects of an antidepressant were not obtained or a depression patient(s) that has (have) not received treatment by said antidepressant, based on the data, the subject may be determined to have obtained the effects of the antidepressant, or may be determined to have a high probability of having obtained the effects of the antidepressant. This is because the sialidase activity in a sample acquired from a healthy individual or a depression patient in whom effects of an antidepressant have been obtained is lower or tends to be lower than the sialidase activity in a sample acquired from a depression patient in whom effects of an antidepressant were not obtained or a depression patient that has not received treatment by said antidepressant.

In another embodiment, the data collection method for evaluating the efficacy of an antidepressant in a subject may be a method that includes implementing the above [Detection method] in the present embodiment on multiple samples acquired over time from the same subject.

According to this method, in the case in which the biomarker measurement values in the samples acquired from the subject exhibit a change at a certain time compared to before that time, based on the data, the subject may be determined to have obtained the effects of the antidepressant, or to have a high probability of having obtained the effects of the antidepressant. By using this method to periodically monitor depression patients during treatment with an antidepressant, the efficacy of the antidepressant can be evaluated and/or alleviation of depression can be evaluated.

In one embodiment, in the case in which the biomarker measurement values in the samples acquired from the subject decrease at a certain time compared to before that time, based on the data, the subject may be determined to have obtained the effects of the antidepressant, or to have a high probability of having obtained the effects of the antidepressant, or the depression may be determined to have been alleviated by the antidepressant, or to have a high probability of having been alleviated. In one embodiment, in the case in which the biomarker measurement values in the samples acquired from the subject increase at a certain time compared to before that time, based on the data, it may be determined that the efficacy of the antidepressant in the subject has decreased or that there is a high probability of the efficacy having decreased, or it may be determined that the depression worsened or that there is a high probability of the depression having worsened. This is because the sialidase activity in a sample acquired from a healthy individual or a depression patient in whom effects of an antidepressant have been obtained is lower or tends to be lower than the sialidase activity in a sample acquired from a depression patient in whom effects of an antidepressant were not obtained or a depression patient that has not received treatment by said antidepressant.

In one embodiment, the efficacy of an antidepressant in a subject can be evaluated by any one of the above-mentioned determinations.

In this case, the reference value may be the mean value or the median value of biomarker measurement values in samples acquired from subjects in the same group or acquired over time from the same subject, or may be a reference range or a normal range that has been suitably set based on biomarker measurement values.

Additionally, the reference value or the normal value may be decided, as appropriate, by a person skilled in the art to be a value by which the efficacy of an antidepressant in a subject can be determined with a desired accuracy and/or precision based on biomarker measurement values in samples acquired from subjects in the same group or acquired over time from the same subject.

Additionally, the biomarker measurement value in a sample acquired from a subject being the same as a reference value may, for example, mean that there is no significant difference from the reference value or the normal value by a suitable statistical process. The biomarker measurement value in a sample acquired from a subject being different from a reference value may, for example, mean that there is a significant difference from the reference value by a suitable statistical process. The biomarker measurement value in a sample acquired from a subject being elevated in comparison with a reference value may, for example, mean that the biomarker measurement value in the sample acquired from the subject is significantly elevated in comparison with the reference value by a suitable statistical process. Additionally, the biomarker measurement value in a sample acquired from a subject being reduced in comparison with a reference value may, for example, mean that the biomarker measurement value in the sample acquired from the subject is significantly reduced in comparison with the reference value by a suitable statistical process.

The statistical processing method can be selected, as appropriate, in accordance with a method that would be well known to a person skilled in the art.

In one embodiment, the data collection method explained above or the method for evaluating the efficacy of an antidepressant based on said data collection method may be implemented in combination with another data collection method for evaluating the efficacy of an antidepressant, or another method for evaluating the efficacy of an antidepressant.

According to the above-mentioned evaluation method, the acquisition of the sample is non-invasive and does not require the intervention of a specialist, and compared with methods requiring blood sampling or a biopsy, the above-mentioned evaluation method enables more convenient biomarker detection and result acquisition. For example, the subject him/herself can perform the steps from acquiring the sample to detecting the biomarker, and can also acquire an evaluation of the efficacy of an antidepressant.

Additionally, by conveniently measuring and detecting biomarkers, objective and/or quantitative evaluation results can be acquired.

### [Kit]

The kit in one embodiment is a kit for evaluating the efficacy of an antidepressant in a subject, the kit including means for detecting any one of the biomarkers described in the above [Biomarker] section in the present embodiment.

In one embodiment, said kit is a kit for implementing a detection method described in the above [Detection method] section in the present embodiment.

Examples and preferred examples of the "kit" are as described in the [Kit] section in the first embodiment.

According to the above-mentioned kit, the acquisition of the sample is non-invasive and does not require the intervention of a specialist, and compared with methods requiring blood sampling or a biopsy, the above-mentioned kit enables more convenient biomarker detection and result acquisition. For example, the subject him/herself can perform the steps from acquiring the sample to detecting the biomarker, and can also acquire an evaluation of the efficacy of an antidepressant.

Additionally, by conveniently measuring and detecting biomarkers, objective and/or quantitative evaluation results can be acquired.

### == Fourth Embodiment (stress level determination marker, bacterial species composition) ==

### [Biomarker]

The biomarker according to the fourth embodiment is the composition of bacterial species in a bacterial population having sialic acid in a sample acquired from a subject. The biomarker of the present embodiment can be used to determine a stress level received by a subject.

Sialic acid is a collective name for the nine-carbon sugar, 2-keto-3-deoxynononic acid, having a carboxyl group at the carbon-1 position, or a collective name for N- and/or O-acyl derivatives having neuraminic acid as the core structure. In the present specification, sialic acid includes sialic acids in both the narrow sense and the broad sense, including N-acetylneuraminic acid (Neu5Ac), N-glycolylneuraminic acid (Neu5Gc), deaminoneuraminic acid (Kdn), etc. The sialic acid is also not limited by, for example, modifications thereof, and may be an O-acetylate, an O-sulfate, a lactone, or a lactam. That is, the bacterial population having sialic acid in the present embodiment may be a bacterial population comprising any of these sialic acids.

The bacterial population having sialic acid preferably has two or more bacteria having cell surface glycans that are sialylated.

In the fourth embodiment, the composition of bacterial species in the phylum *Firmicutes* (*p_Firmicutes*) in the bacterial population in a sample acquired from a subject can also be used to determine a stress level received by a subject.

The bacterial population preferably has two or more bacteria.

### (Stress level)

The "stress level" in the present embodiment is as described in the "Stress level" portion of the [Biomarker] section in the first embodiment.

### (Sample)

The sample is not limited as long as it can be acquired from a subject, but is preferably a sample from the gut of the subject. By using, as a biomarker, the composition of bacterial species in a bacterial population having sialic acid contained in a sample from the gut or the composition of bacterial species in the phylum *Firmicutes* (*p_Firmicutes*) in a bacterial population contained in a sample from the gut, the stress level received by a subject can be easily determined with high accuracy and/or precision. As samples from the gut, there are, for example, stool and intestinal mucosal tissue.

In this case, the stool is not limited as long as it includes biological samples from the gut of a subject, and broadly includes gut contents. For example, it includes both gut contents after they have been excreted outside the body of the subject (so-called "stool") and gut contents before excretion. From the aspect of being non-invasive and not requiring the intervention of a specialist when acquiring the sample, the sample from the gut is preferably stool excreted outside the body of the subject.

The region of the gut from which the gut contents come is not limited, and for example, may be the small intestine including the duodenum, the jejunum, and the ileum, the large intestine including the cecum, the appendix, the colon, and the rectum, etc. In this case, the large intestine covers, for example, the ascending colon, the transverse colon, the descending colon, the sigmoid colon, the upper rectum, the lower rectum, etc.

If the sample is stool, it should be sampled and stored in accordance with technology that would be well known to a person skilled in the art so as to suppress the intermixture of undesired matter that is not originally contained in the stool and/or so that the composition of bacterial species in the stool does not change after excretion from the subject and until the composition of bacterial species is detected.

### (Composition of bacterial species)

The composition of bacterial species, which is the biomarker in the present embodiment, is the composition of bacterial species in a bacterial population having sialic acid in a sample acquired from a subject or the composition of bacterial species in the phylum *Firmicutes* (*p_Firmicutes*) in a bacterial population in a sample acquired from a subject. Considering the accuracy and/or precision of the determination, the bacteria are preferably gut bacteria that live in the gut of the subject.

Since there is a possibility that a sample from the gut contains microbes other than bacteria, the "bacterial population having sialic acid" may include microbes having sialic acid other than bacteria. However, considering the accuracy and/or precision of the determination, the "bacterial population having sialic acid" should preferably not include microbes other than bacteria. The bacterial population having sialic acid may be all bacteria having sialic acid among the bacteria included in a sample, or may be some of the bacteria having sialic acid. In this case, a bacterial population having sialic acid refers to a bacterial population having cell surface glycans that are sialylated. The cell surface glycans are not limited and may, for example, be lipopolysaccharides, lipoteichoic acid, capsular polysaccharides, etc. that are modified by sialic acid.

Samples acquired from a subject normally include multiple bacterial species. For example, human gut bacteria are considered to include 1000 or more species. The "composition of bacterial species in a bacterial population having sialic acid" is the amount of one or more suitable bacterial species contained in a bacterial population having sialic acid.

In this case, the "amount contained" may, for example, be the mass or the bacterial cell count. Additionally, the "amount contained" may, for example, be an absolute value or a relative value of the amount of one or more suitable bacterial species contained in the bacterial population having sialic acid. In the case of an absolute value, it may, for example, be the mass of one or more bacterial species in the bacterial population having sialic acid of a suitable mass, or may be the bacterial cell count of one or more bacterial species in the bacterial population having sialic acid. In the case of a relative value, it may, for example, be the ratio of the mass or the bacterial cell count of one or more suitable bacterial species to the mass or the bacterial cell count of the bacterial population having sialic acid, or may be the percentage of the mass or the bacterial cell count of two or more suitable bacterial species in the bacterial population having sialic acid.

In the present specification, "bacterial species" is not limited to meaning only the "species" of the bacteria in terms of biological taxonomy, but refers more broadly to the type of bacteria. For example, if the composition being detected is a composition by bacterial class, order, or genus, then the bacteria belonging to those classifications are meant.

Additionally, the "composition of bacterial species in the phylum *Firmicutes* (*p_Firmicutes*) in a bacterial population" is the amount of one or more bacterial species belonging to the phylum *Firmicutes* (*p_Firmicutes*) contained in a bacterial population.

In this case, the "amount contained" may, for example, be the mass or the bacterial cell count. Additionally, the "amount contained" may, for example, be an absolute value or a relative value of the amount of one or more suitable bacterial species contained in the bacterial population. In the case of an absolute value, it may, for example, be the mass of one or more bacterial species belonging to the phylum *Firmicutes* (*p_Firmicutes*) in the bacterial population, or may be the bacterial cell count of one or more bacterial species belonging to the phylum *Firmicutes* in the bacterial population. In the case of a relative value, it may, for example, be the ratio of the mass or the bacterial cell count of one or more bacterial species belonging to the phylum *Firmicutes* (*p_Firmicutes*) to the mass or the bacterial cell count of the bacterial population, or may be the percentage of the mass or the bacterial cell count of two or more bacterial species belonging to the phylum *Firmicutes* (*p_Firmicutes*) in the bacterial population.

In the present specification, "bacterial species" is not limited to meaning only the "species" of the bacteria in terms of biological taxonomy, but refers more broadly to the type of bacteria. For example, if the composition being detected is a composition by bacterial class, order, or genus, then the bacteria belonging to those classifications are meant.

The bacterial species in the bacterial population having sialic acid are not limited as long as the composition of those bacterial species in the bacterial population having sialic acid is correlated with the stress level received by a subject. However, they preferably include one bacterial species or a combination of two or more bacterial species in the class *Clostridia* (*c_Clostridia*), the order *Clostridiales (o_Clostridiales),* the genus *Ruminococcus* (*g_Ruminococcus*), the genus *Lactobacillus* (*g_Lactobacillus*), the family *Erysipelotrichaceae* (*f_Erysipelotrichaceae*), *Lactobacillus reuteri* (*g_Lactobacillus s_reuteri*)*,* the order *Lactobacillales (o_Lactobacillales),* and the species *Erysipelotrichaceae (s_Erysipelotrichaceae).* They preferably include one bacterial species or a combination of two or more bacterial species in the class *Clostridia* (*c_Clostridia*), the family *Erysipelotrichaceae* (*L_Erysipelotrichaceae*), the genus *Lactobacillus* (*g_Lactobacillus*), and the species *Erysipelotrichaceae* (*s_Erysipelotrichaceae*). They more preferably include bacteria in the class *Clostridia (c_Clostridia).*

For example, in the case in which the bacterial species in the bacterial population having sialic acid is bacteria in the class *Clostridia* (*c_Clostridia*)*,* the composition thereof in the bacterial population having sialic acid can be used as a biomarker whether the bacteria in the class *Clostridia* (*c_Clostridia*) are just a portion of the bacterial species in the class *Clostridia* (*c_Clostridia*) contained in the sample, or all bacterial species in the class *Clostridia* (*c_Clostridia*) contained in the sample. For example, it is possible to use, as a biomarker, the composition of bacteria in the order *Clostridiales* (*o_Clostridiales*) and/or other orders, or of bacteria in the genus *Clostridium* (*g_Clostridium*) and/or other genera, among the bacteria in the class *Clostridia* (*c_Clostridia*) in the bacterial population having sialic acid.

In one embodiment, the composition of the bacterial species in the bacterial population having sialic acid is preferably the amount of bacteria in the class *Clostridia* (*c_Clostridia*) contained in a bacterial population having sialic acid.

The bacterial species in the phylum *Firmicutes* in the bacterial population are not limited as long as the composition of those bacterial species belonging to the phylum *Firmicutes* (*p_Firmicutes*) is correlated with the stress level received by a subject. They may, for example, be all bacterial species belonging to the phylum *Firmicutes* (*p_Firmicutes*)*,* or bacterial species belonging to the respective classes in the phylum *Firmicutes.* Preferably, they are all bacterial species belonging to the phylum *Firmicutes* (*p_Firmicutes*), or bacterial species in the class *Clostridia*

### (c_Clostridia).

In this case, it is known, regarding the microbiota in stool, that the relative amount of *Clostridium* (*g_Clostridium*) is approximately the same in humans and in mice (Thi Loan Anh Nguyen et al., "How informative is the mouse for human gut microbiota research?", Disease Models & Mechanisms, Vol. 8 (1), Jan 2015, pages 1-16).

### (Subject)

Examples and preferred examples of the "subject" are as described in the "Subject" portion of the [Biomarker] section in the first embodiment, with the exceptions below.

In one embodiment, the biomarker for determining a stress level received by a subject according to the present embodiment is preferably the composition of bacterial species in a bacterial population having sialic acid in a sample acquired from a human.

In one embodiment, the biomarker for determining a stress level received by a subject according to the present embodiment is more preferably the composition of bacterial species in a bacterial population having sialic acid in stool acquired from a human.

In one embodiment, the biomarker for determining a stress level received by a subject according to the present embodiment is even more preferably the amount of bacteria in the class *Clostridia* (*c_Clostridia*) contained in stool acquired from a human.

In one embodiment, the biomarker for determining a stress level received by a subject according to the present embodiment is preferably the composition of bacterial species belonging to the phylum *Firmicutes* (*p_Firmicutes*) in a bacterial population in a sample acquired from a human.

In one embodiment, the biomarker for determining a stress level received by a subject according to the present embodiment is more preferably the composition of bacterial species belonging to the phylum *Firmicutes* (*p_Firmicutes*) in a bacterial population in stool acquired from a human.

### [Detection method]

The detection method in one embodiment is a biomarker detection method for detecting any one of the biomarkers described in the above [Biomarker] section in the present embodiment in a sample acquired from a subject.

Due to the detection method described below, any one of the biomarkers described in the above [Biomarker] section in the present embodiment can be detected in a sample acquired from a subject.

In this case, regarding the examples and the preferred examples of the "sample", the "composition of bacterial species", and the "subject", they are as described in the above [Biomarker] section in the present embodiment.

The sample is acquired from the subject as described in the [Detection method] section in the first embodiment.

Regarding pretreatments preceding biomarker detection, they can be performed on a sample acquired from a subject as described in the [Detection method] section in the first embodiment, with the exceptions below.

In the case in which the biomarker according to the present embodiment is the composition of bacterial species in a bacterial population having sialic acid, the detection method according to the present embodiment may include a step of isolating a bacterial population having sialic acid from a sample acquired from a subject. The bacterial population having sialic acid can be isolated from a sample by using, for example, a lectin that specifically binds to a sialylated glycan. The lectin that is used can be selected, as appropriate, by a person skilled in the art in accordance with the type of sialylated glycan to be bound. For example, lectins such as *Sambucus nigra* lectin (elderberry) bark (SNA), *Limax flavus* lectin (LFA), *Triticum vulgaris* lectin (WGA), *Maackia amurensis* lectin I (MAL I), *Maackia amurensis* lectin II (MAL II), *Agrocybe cylindracea* (ACG), galectin 8 N-terminal domain (Gal 8 N), *Sambucus sieboldiana* lectin (SSA), *Trichosanthes japonica* lectin (TJAI), *Polyporus squamosus* lectin 1a (rPSL1a), *Allomyrina dichtoma* lectin (ADA), *Escherichia coli* lectin (SubB2M), *Salmonella enterica* lectin (PItB), *Streptococcus gordonii* lectin (HAS), and porcine hemagglutinating encephalomyelitis virus lectin (BCoV) can be used. Information regarding other lectins is available from the lectin frontier database (LfDB), from Odaka et al. (STAR Protoc., 2022 Feb 18; 3(1):101179. doi: 10.1016/j.xpro.2022.101179), or from Minoshima et al. (iScience. 2021 Jul 17; 24(8):102882. doi: 10.1016/j.isci.2021.102882).

In the case in which the biomarker according to the present embodiment is the composition of bacterial species in a bacterial population, the "bacterial population" may not have undergone a step of isolating the bacterial population according to the presence or absence of sialic acid for a sample acquired from a subject. The composition of bacterial species in all bacterial populations contained in a sample can be used as the biomarker.

The detection of the biomarker is not limited as long as the composition of bacterial species in the bacterial population having sialic acid or the composition of bacterial species belonging to the phylum *Firmicutes* (*p_Firmicutes*) in the bacterial population is detected. As described in the above [Biomarker] section in the present embodiment, the composition of bacterial species in the bacterial population having sialic acid may be an absolute value or a relative value of the amount of one or more suitable bacterial species contained in the bacterial population having sialic acid. Additionally, the composition of bacterial species belonging to the phylum *Firmicutes* (*p_Firmicutes*) in the bacterial population may be an absolute value or a relative value of the amount of one or more suitable bacterial species belonging to the phylum *Firmicutes* (*p_Firmicutes*) contained in the bacterial population.

In the present specification, the composition of the biomarker (the composition of bacterial species in a bacterial population or the composition of specific bacterial species in a bacterial population) is also referred to as the biomarker measurement value, and may be any one of the presence or absence of a biomarker in the sample, an absolute value, and a relative value.

In this case, the composition of bacterial species in the bacterial population having sialic acid or the composition of bacterial species belonging to the phylum *Firmicutes* (*p_Firmicutes*) in the bacterial population can be detected, for example, by a method that includes directly measuring the amount of the bacterial cells that are contained, or measuring a suitable physical amount, derived from the bacterial cells, correlated with the amount of the bacterial cells that are contained.

In the case in which the physical amount of the bacterial cells is to be directly measured, for example, the mass of the bacterial population having sialic acid or of the bacterial population and the mass of each of one or more suitable bacterial species contained in the bacterial population can be measured. In the case in which the cell count of the bacterial cells is to be directly measured, for example, the number of bacterial cells constituting the bacterial population having sialic acid or the bacterial population, and the number of bacterial cells of each of one or more suitable bacterial species contained in the bacterial population can be counted. Due to these measurements, the amount of the one or more suitable bacterial species contained in the bacterial population having sialic acid, or the absolute value of the composition of one or more bacterial species belonging to the phylum *Firmicutes* (*p_Firmicutes*) in the bacterial population can be acquired.

Additionally, the measurement values acquired by these measurements can be used to detect the composition of bacterial species by calculating the ratio of the mass or the bacterial cell count of the one or more suitable bacterial species with respect to the mass or the bacterial cell count of the bacterial population having sialic acid. Alternatively, the composition of bacterial species can be detected by calculating, for example, the percentage of the mass or the bacterial cells of two or more suitable bacterial species in the bacterial population having sialic acid.

In the case in which a suitable physical amount derived from the bacterial cells and correlated with the bacterial cell count is to be measured, for example, a suitable physical amount correlated with the mass of the cell population having sialic acid, or correlated with the number of bacterial cells constituting the bacterial population having sialic acid, and a suitable physical amount correlated with the mass or the bacterial cell count of each of the one or more suitable bacterial species contained in the bacterial population can be measured. By using these measurement values also, as in the case in which absolute values are used, the composition of bacterial species can be detected by calculating the ratio of the one or more suitable bacterial species with respect to the bacterial population having sialic acid. Alternatively, for example, the composition of bacterial species can be detected by calculating the percentage of two or more suitable bacterial species in the bacterial population having sialic acid.

In the case in which the biomarker is the composition of bacterial species in the phylum *Firmicutes* (*p_Firmicutes*) in a bacterial population also, the composition of the bacterial species can be similarly detected by calculating the ratio of the mass or the bacterial cell count of one or more bacterial species belonging to the phylum *Firmicutes* (*p_Firmicutes*) in the bacterial population. Alternatively, for example, the composition of bacterial species can be detected by calculating the percentage of the mass or the bacterial cells of two or more bacterial species belonging to the phylum *Firmicutes* (*p_Firmicutes*) in the bacterial population.

In the case in which the cell count of bacterial cells is to be directly measured, for example, a bacteria counter may be used to count the bacterial cells under a microscope, an agar culture medium may be inoculated and the number of colonies counted, a liquid culture medium may be inoculated and the transmitted light intensity may be measured with a spectrophotometer and compared with a reference that has been acquired in advance to count the number of bacterial cells, or the weight of the bacteria may be measured and compared with a reference that has been acquired in advance to count the number of bacterial cells.

In the case in which a suitable physical amount correlated with the bacterial cell count is to be measured, for example, the amount of ATP from bacteria may be measured, the amount of nucleic acid fragments from bacteria may be measured, sialic acid from the cell population having sialic acid may be measured, or these measurement values may be compared with a reference that has been acquired in advance to count the number of bacterial cells. Alternatively, the glycan sequencing (Glycan-seq) method developed by Oinam *et al.* may be used to measure specific bacteria having sialic acid by causing bacteria having sialic acid to bind to lectins labeled with different DNA for each sialylated glycan thereof and detecting the DNA by means of a method using a primer or a probe.

In the case in which the amount of nucleic acid fragments from bacteria is to be measured, for example, it is possible to use a method using different primers or probes for nucleic acid sequences specific to some or all of specific bacterial populations such as the class *Clostridia* (*c_Clostridia*), the order *Clostridiales* (*o_Clostridiales*), the genus *Ruminococcus* (*g*_*Ruminococcus*), the genus *Lactobacillus* (*g_Lactobacillus*), the family *Erysipelotrichaceae* (*f_Erysipelotrichaceae*), *Lactobacillus reuteri* (*g_Lactobacillus s_reuteri*), the order *Lactobacillales* (*o_Lactobacillales*), the species *Erysipelotrichaceae* (*s_Erysipelotrichaceae*), and the phylum *Firmicutes* (*p_Firmicutes*). The method can be selected, as appropriate, by a person skilled in the art, and for example, includes specific quantitative PCR, terminal-restriction fragment-length polymorphism (T-RFLP), fluorescence in-situ hybridization (FISH), or a method using a microarray, etc., a clone library method, denaturing/temperature gradient gel electrophoresis (DGGE/TGGE), metagenomic analysis, etc.

More specifically, in the case of using a method using a different primer or probe for each of the one or more bacterial species contained in a bacterial population having sialic acid or in the case of using a method using a different primer or probe for each of the one or more bacterial species belonging to the phylum *Firmicutes* (*p_Firmicutes*) contained in a bacterial population, for example, nucleic acids can be amplified by PCR, etc. using primers specific to the respective bacterial species, and the amount of nucleic acids can be measured during the amplification reaction or after the amplification reaction, as in specific quantitative PCR or T-RFLP. Alternatively, the nucleic acids can be detected using a probe, etc. specific to the genus of the bacteria, without amplifying the nucleic acids, as in a method using FISH or a microarray. The primers and the probes that are used may target corresponding regions on the nucleic acids of the respective bacterial species, or may target different regions. Specifically, for example, in the case of using a 16S rRNA sequence, for example, some or all of nine variable regions can be used as targets. More specifically, for example, the V3-V4 hypervariable region in the 16S rRNA gene may be targeted and the region may be specifically amplified by a PCR reaction.

Normally, a sequencer is used for a method using a primer that is common to some or all of the one or more bacterial species contained in a bacterial population having sialic acid or for a method using a primer that is common to some or all of the one or more bacterial species belonging to the phylum *Firmicutes* (*p_Firmicutes*) contained in a bacterial population. The treatment performed on the nucleic acids before sequencing may be selected, as appropriate, by a person skilled in the art. For example, in the clone library method, sequence portions specific to some or all of the bacterial species are amplified from extracted nucleic acids, and the amplification products thereof are inserted in plasmids for sequencing. In DGGE/TGGE, an amplification product that has been similarly amplified is separated in accordance with the sequence of the nucleic acid chain by means of electrophoresis, then extracted from a gel and sequenced. In metagenomic analysis, the nucleic acids are fragmented or amplified, and the products thereof are directly sequenced. The primers used for amplification and sequencing may be the same primer or different primers. For example, in the case of using a 16S rRNA sequence, primers that are hybridizable to, for example, conserved regions can be used. As such primers, a forward primer having the nucleic acid sequence of SEQ ID NO:83 and a reverse primer having the nucleic acid sequence of SEQ ID NO:84 may be used. For sequencing, a sequencer using the principles of the Sanger method or a next-generation sequencer can be used. As sequencing methods, for example, there are multiplex sequencing, single-read sequencing, paired-end sequencing, etc., which can be selected, as appropriate, by a person skilled in the art.

The method for measuring the suitable physical amount correlated with the bacterial cell count described above can be performed by using, for example, a commercially available kit.

In the case in which sialic acid from a cell population having sialic acid is to be measured, this can be performed, as appropriate, in accordance with methods that would be well known to a person skilled in the art. For example, sialic acid-modified glycans can be decomposed to monosaccharides, the amount of sialic acid may be quantified and the absolute amount investigated by using liquid chromatography or mass spectrometry.

The method for identifying bacterial species from sequence information obtained by sequencing is not particularly limited. For example, an analysis method that is known in the relevant technical field, making use of analysis software, databases, etc., can be used. In the case in which a database is used, for example, bacterial species can be identified by performing sequency identity searches on collected bacterial base sequences. Specific examples of databases include GenBank, ENA, DDBJ, etc. Additionally, the GREENGENES database, which is a database specializing in 16S rRNA sequences, etc. may be used. Alternatively, a tool for microbe analysis, such as the amplicon sequence data analysis software QIIME 2 (Bolyen E. et al., "Reproducible, interactive, scalable and extensible microbiome data science using QIIME 2", Nature Biotechnology 37: 852-857, 2019, doi.org/10.1038/s41587-019-0209-9), etc. can be used to identify the bacterial species.

According to the above-mentioned detection method, the acquisition of the sample is non-invasive. Additionally, by measuring and detecting biomarkers, objective and/or quantitative test results can be acquired.

### [Determination method]

The data collection method in one embodiment is a data collection method for determining a stress level received by a subject, including a detection method described in the above [Detection method] section in the present embodiment.

In one embodiment, the stress level received by a subject can be determined based on data collected by said data collection method. That is, the method for determining a stress level received by a subject according to one embodiment includes said data collection method.

The detection of the composition of bacterial species in a bacterial population having sialic acid, which is a biomarker, can be performed by means of any one of the detection methods described in the above [Detection method] section in the present embodiment.

The detection of the composition of bacterial species belonging to the phylum *Firmicutes* (*p_Firmicutes*) in a bacterial population in a sample acquired from a subject, which is a biomarker, can be performed by means of any one of the detection methods described in the above [Detection method] section in the present embodiment.

In one embodiment, the data collection method for determining a stress level received by a subject may be a method that includes, before implementing any one of the detection methods described in the above [Detection method] section in the present embodiment on a sample acquired from a subject, implementing a biomarker detection method according to the above [Detection method] section in the present embodiment on a sample(s) acquired from one or more individuals that have received a prescribed level of stress, and acquiring a biomarker reference value for an individual(s) that has (have) received the prescribed level of stress.

In another embodiment, the data collection method for determining a stress level received by a subject may be a method that includes, before implementing any one of the detection methods described in the above [Detection method] section in the present embodiment on a sample acquired from a subject, implementing a biomarker detection method according to the above [Detection method] section in the present embodiment on a sample(s) acquired from one or more individuals that have received a prescribed level of stress and a sample(s) acquired from one or more individuals that have not received the prescribed level of stress, and acquiring a biomarker reference value for an individual(s) that has (have) received the prescribed level of stress and a biomarker reference value (normal value) for an individual(s) that has (have) not received the prescribed level of stress.

According to these methods, in the case in which a biomarker measurement value in a sample acquired from a subject is the same as a biomarker reference value for an individual(s) that has (have) received a prescribed level of stress, based on the data, the subject may be determined to have received or to be receiving the prescribed level of stress, or may be determined to have a high probability of having received or receiving the prescribed level of stress. Alternatively, in the case in which a biomarker measurement value in a sample acquired from a subject is different from a biomarker reference value for an individual(s) that has (have) received a prescribed level of stress, based on the data, the subject may be determined to not have received the prescribed level of stress, or may be determined to have a high probability of not having received the prescribed level of stress.

Additionally, in the case in which a biomarker measurement value in a sample acquired from a subject is different from a normal value, based on the data, the subject may be determined to have received or to be receiving a prescribed level of stress, or may be determined to have a high probability of having received or receiving a prescribed level of stress. Alternatively, in the case in which a biomarker measurement value in a sample acquired from a subject is the same as a normal value, based on the data, the subject may be determined to not have received a prescribed level of stress or may be determined as having a high probability of not having received a prescribed level of stress.

In one embodiment, in the case in which a biomarker measurement value in a sample acquired from a subject is elevated in comparison with a normal value, based on the data, the subject may be determined to have received or to be receiving a prescribed level of stress, or may be determined to have a high probability of having received or receiving a prescribed level of stress.

In individuals that have received a prescribed stress, the amount of bacteria in the class *Clostridia* (*c_Clostridia*) contained in a bacterial population having sialic acid in a sample is high or tends to be high in comparison with individuals that have not received stress. Therefore, in the case in which the amount of bacteria in the class *Clostridia* (*c_Clostridia*) contained in a sample acquired from a subject is high, the subject may be determined to have a high probability of having received or receiving a prescribed level of stress.

In individuals that have received a prescribed stress, the amount of bacteria in the order *Clostridiales* (*o_Clostridiales*) contained in a bacterial population having sialic acid in a sample is high or tends to be high in comparison with individuals that have not received stress. Therefore, in the case in which the amount of bacteria in the order *Clostridiales* (*o_Clostridiales*) contained in a sample acquired from a subject is high, the subject may be determined to have a high probability of having received or receiving a prescribed level of stress.

In individuals that have received a prescribed stress, the amount of bacteria in the genus *Ruminococcus* (*g_Ruminococcus*) contained in a bacterial population having sialic acid in a sample is high or tends to be high in comparison with individuals that have not received stress. Therefore, in the case in which the amount of bacteria in the genus *Ruminococcus* (*g_Ruminococcus*) contained in a sample acquired from a subject is high, the subject may be determined to have a high probability of having received or receiving a prescribed level of stress.

In individuals that have received a prescribed stress, the amount of bacteria in the family *Erysipelotrichaceae* (*f_Erysipelotrichaceae*) contained in a bacterial population having sialic acid in a sample is low or tends to be low in comparison with individuals that have not received stress. Therefore, in the case in which the amount of bacteria in the family *Erysipelotrichaceae* (*f_Erysipelotrichaceae*) contained in a sample acquired from a subject is low, the subject may be determined to have a high probability of having received or receiving a prescribed level of stress.

In individuals that have received a prescribed stress, the amount of bacteria in the genus *Lactobacillus* (*g_Lactobacillus*) contained in a bacterial population having sialic acid in a sample is low or tends to be low in comparison with individuals that have not received stress. Therefore, in the case in which the amount of bacteria in the genus *Lactobacillus* (*g*_*Lactobacillus*) contained in a sample acquired from a subject is low, the subject may be determined to have a high probability of having received or receiving a prescribed level of stress.

In individuals that have received a prescribed stress, the amount of the bacteria *Lactobacillus reuteri* (*g_Lactobacillus s_reuteri*) contained in a bacterial population having sialic acid in a sample is low or tends to be low in comparison with individuals that have not received stress. Therefore, in the case in which the amount of the bacteria *Lactobacillus reuteri* (*g_Lactobacillus s_reuteri*) contained in a sample acquired from a subject is low, the subject may be determined to have a high probability of having received or receiving a prescribed level of stress.

In individuals that have received a prescribed stress, the amount of bacteria in the order *Lactobacillales* (*o_Lactobacillales*) contained in a bacterial population having sialic acid in a sample is low or tends to be low in comparison with individuals that have not received stress. Therefore, in the case in which the amount of bacteria in the order *Lactobacillales* (*o_Lactobacillales*) contained in a sample acquired from a subject is low, the subject may be determined to have a high probability of having received or receiving a prescribed level of stress.

In individuals that have received a prescribed stress, the amount of bacteria of the species *Erysipelotrichaceae* (*s_Erysipelotrichaceae*) contained in a bacterial population having sialic acid in a sample is low or tends to be low in comparison with individuals that have not received stress. Therefore, in the case in which the amount of bacteria of the species *Erysipelotrichaceae* (*s_Erysipelotrichaceae*) contained in a sample acquired from a subject is low, the subject may be determined to have a high probability of having received or receiving a prescribed level of stress.

In individuals that have received a prescribed stress, the amount of bacteria in the phylum *Firmicutes* (*p_Firmicutes*) contained in a bacterial population in a sample is high or tends to be high in comparison with individuals that have not received stress. Therefore, in the case in which the amount of bacteria in the phylum *Firmicutes* (*p_Firmicutes*) contained in a sample acquired from a subject is high, the subject may be determined to have a high probability of having received or receiving a prescribed level of stress.

In individuals that have received a prescribed stress, the amount of bacteria in the class *Clostridia* (*c_Clostridia*) contained in a bacterial population in a sample is high or tends to be high in comparison with individuals that have not received stress. Therefore, in the case in which the amount of bacteria in the class *Clostridia* (*c_Clostridia*) contained in a sample acquired from a subject is high, the subject may be determined to have a high probability of having received or receiving a prescribed level of stress.

In another embodiment, the data collection method for determining a stress level received by a subject may be a method that includes, before implementing any one of the detection methods described in the above [Detection method] section in the present embodiment on a sample acquired from a subject, implementing a biomarker detection method according to the above [Detection method] section in the present embodiment on samples acquired from multiple individuals that have received respectively different levels of stress or samples from multiple groups of individuals that have received respectively different levels of stress, and setting a standard curve indicating the relationship between stress levels and biomarker levels based on the biomarker levels in the individuals or the groups of individuals that have received different levels of stress.

According to this method, in the case in which a biomarker measurement value in a sample acquired from a subject is the same as any point lying inside the standard curve, based on the data, the subject may be determined to have received or to be receiving stress at a level corresponding to said point, or may be determined to have a high probability of having received or receiving the prescribed level of stress. In the case in which a biomarker measurement value in a sample acquired from a subject is different from any of the points lying inside the standard curve, based on the data, the subject may be determined to not have received stress at a level corresponding to any of the points lying inside the standard curve, or may be determined to have a high probability of not having received stress at a level corresponding to any of the points lying inside the standard curve.

In another embodiment, the data collection method for determining a stress level received by a subject may be a method that includes implementing any one of the detection methods described in the above [Detection method] section in the present embodiment on multiple samples acquired over time from the same subject.

According to this method, in the case in which the biomarker measurement values in the samples acquired from the subject exhibit a change at a certain time compared to before that time, based on the data, the subject may be determined to have received or to be receiving a prescribed level of stress, or to have a high probability of having received or receiving a prescribed level of stress. For example, through periodically implemented health checkups, etc., this method can be used to determine and to monitor stress levels received or being received by a subject.

In one embodiment, in the case in which the biomarker measurement values in the samples acquired from the subject increase at a certain time compared to before that time, based on the data, the subject may be determined to have received or to be receiving a prescribed level of stress, or to have a high probability of having received or receiving a prescribed level of stress. For example, in the case in which the biomarker, which is the composition of bacterial species in a bacterial population having sialic acid, is the amount of bacteria in the class *Clostridia (c_Clostridia)* that is contained, this is because the amount of bacteria in the class *Clostridia (c_Clostridia)* contained in a bacterial population having sialic acid in a sample is high or tends to be high in individuals that have received stress, or in individuals that have received stress and that have been administered an antidepressant, in comparison with individuals that have not received stress.

In one embodiment, the stress level received by a subject can be determined by any one of the above-mentioned determinations.

In this case, the reference value, the normal value, or the standard curve may be set based on the mean value or the median value of biomarker measurement values in samples acquired from individuals in the same group or acquired over time from the same individual, or may be a reference range, a normal range, or a standard curve that has been suitably set based on biomarker measurement values.

Alternatively, the reference value, the normal value, or the standard curve may be decided, as appropriate, by a person skilled in the art to be a value by which the stress level received by a subject can be determined with a desired accuracy and/or precision based on biomarker measurement values in samples acquired from individuals in the same group or acquired over time from the same individual.

Additionally, the biomarker measurement value in a sample acquired from a subject being the same as a reference value, a normal value, or any point lying inside a standard curve, for example, means that there is no significant difference from the reference value or the normal value by a suitable statistical process. The biomarker measurement value in a sample acquired from a subject being different from a reference value, a normal value, or a point lying inside a standard curve, for example, means that there is a significant difference from the reference value, the normal value, or the point lying inside the standard curve by a suitable statistical process. The biomarker measurement value in a sample acquired from a subject being elevated in comparison with a reference value, for example, means that the biomarker measurement value in the sample acquired from the subject is significantly elevated in comparison with the reference value by a suitable statistical process.

The statistical processing method can be selected, as appropriate, in accordance with a method that would be well known to a person skilled in the art.

In one embodiment, the reference value may be a value that is 150%, 200%, 250%, 300%, 350%, 400%, 450%, or 500% or more with respect to the normal value, or may be decided, as appropriate, by a person skilled in the art to be a value by which the stress level received by a subject can be determined with a desired precision.

In one embodiment, the data collection method explained above or the method for determining a stress level received by a subject based on said data collection method may be implemented in combination with another data collection method for determining a stress level received by a subject, or another method for determining a stress level received by a subject.

### [Kit]

The kit in one embodiment is a kit for determining a stress level received by a subject, the kit including means for detecting any one of the biomarkers described in the above [Biomarker] section in the present embodiment.

In one embodiment, said kit is a kit for implementing a detection method described in the above [Detection method] section in the present embodiment.

In one embodiment, in the case in which the kit is for implementing a detection method for detecting the composition of bacterial species in a bacterial population having sialic acid, the kit includes, for example, means for isolating a bacterial population having sialic acid in a sample acquired from a subject. As such means, for example, there are lectins that specifically bind to sialylated glycans. The lectins may be supported on a suitable support so as to be adapted for isolation of a target cell population. As such lectins, for example, *Sambucus nigra* lectin (elderberry) bark (SNA), *Limax flavus* lectin (LFA), *Triticum vulgaris* lectin (WGA), *Maackia amurensis* lectin I (MAL I), *Maackia amurensis* lectin II (MAL II), *Agrocybe cylindracea* (ACG), galectin 8 N-terminal domain (Gal 8 N), *Sambucus sieboldiana* lectin (SSA), *Trichosanthes japonica* lectin (TJAI), *Polyporus squamosus* lectin 1a (rPSL1a), *Allomyrina dichtoma* lectin (ADA), *Escherichia coli* lectin (SubB2M), *Salmonella enterica* lectin (PItB), *Streptococcus gordonii* lectin (HAS), porcine hemagglutinating encephalomyelitis virus lectin (BCoV), etc. can be used. Information regarding other lectins is available from the lectin frontier database (LfDB), from Odaka et al. (STAR Protoc., 2022 Feb 18; 3(1):101179. doi: 10.1016/j.xpro.2022.101179), and from Minoshima et al. (iScience. 2021 Jul 17; 24(8):102882. doi: 10.1 016/j.isci.2021.1 02882).

In one embodiment, the kit includes means for directly measuring the cell count of bacterial cells. As such means, there are, for example, bacteria counters, agar culture media or one or more reagents for preparing agar culture media, and/or liquid culture media.

In one embodiment, the kit includes means for measuring a suitable physical amount correlated with the bacterial cell count. For example, it may be means for measuring the amount of ATP, such means including, for example, fluorescent ATP sensors that can bind to ATP to detect the ATP concentration. Additionally, for example, it may be means for measuring the amount of nucleic acid fragments from bacteria, such means including, for example, primers or probes for nucleic acid sequences that are specific to the bacterial populations or the bacterial species to be detected. Other means include lectins, DNA labels, and primers or probes for detecting DNA, used in the glycan sequencing (Glycan-seq) method developed by Oinam *et al.*

The kit may also include a reagent for detecting the composition of bacterial species, a positive control reagent indicating that the detection step is functioning, and/or a storage liquid for storing a sample that has been sampled from a subject until the detection method is implemented, and/or instructions for using the kit.

In one embodiment, the kit may include a detection device necessary for detection, or may be provided together with said detection device.

In one embodiment, at least a portion of said kit may be provided in a state of being installed on a portion of a toilet bowl. By being installed on a toilet bowl, the composition of bacterial species in a bacterial population having sialic acid in stool excreted from a subject can be easily used for health management.

According to the above-mentioned kit, the acquisition of the sample is non-invasive. Additionally, by measuring and detecting biomarkers, objective and/or quantitative test results can be acquired.

### == Fifth Embodiment (depression diagnosis marker, bacterial species composition) ==

### [Biomarker]

The biomarker according to the fifth embodiment is the composition of bacterial species in a bacterial population having sialic acid in a sample acquired from a subject. The biomarker of the present embodiment can be used to diagnose depression in a subject.

In the fifth embodiment, the composition of bacterial species in the phylum *Firmicutes* (*p_Firmicutes*) in a bacterial population in a sample acquired from a subject can also be used for diagnosing depression in a subject.

Depression is known to be caused by stress. Thus, the composition of bacterial species in a bacterial population having sialic acid in a sample acquired from a subject, or the composition of bacterial species of the phylum *Firmicutes* (*p_Firmicutes*) in a bacterial population, which is the biomarker according to the fourth embodiment, can be used to diagnose depression in a subject.

Examples and preferred examples of "depression" are as described in the "Depression" portion of the [Biomarker] section in the second embodiment.

### (Sample)

Examples and preferred examples of the "sample" are as described in the "Sample" portion of the [Biomarker] section in the fourth embodiment.

### (Composition of bacterial species)

Examples and preferred examples of the "composition of bacterial species" are as described in the "Composition of bacterial species" portion of the [Biomarker] section in the fourth embodiment.

### (Subject)

Examples and preferred examples of the "subject" are as described in the "Subject" portion of the [Biomarker] section in the fourth embodiment.

### [Detection method]

The detection method in one embodiment is a biomarker detection method for detecting any one of the biomarkers described in the above [Biomarker] section in the present embodiment in a sample acquired from a subject.

In this case, regarding the examples and the preferred examples of the "sample", the "composition of bacterial species", and the "subject", they are as described in the above [Biomarker] section in the present embodiment.

In the present embodiment, the acquisition of the sample from the subject, the pretreatment preceding biomarker detection, and the detection of the biomarker are as described in the [Detection method] section in the fourth embodiment.

According to the above-mentioned detection method, the acquisition of the sample is non-invasive. Additionally, by measuring and detecting biomarkers, objective and/or quantitative test results can be acquired.

### [Diagnostic method]

The data collection method in one embodiment is a data collection method for diagnosing depression in a subject, including a detection method described in the above [Detection method] section in the present embodiment.

In one embodiment, depression can be diagnosed in a subject based on data collected by said data collection method. That is, the method for diagnosing depression in a subject according to one embodiment includes said data collection method.

The detection of the composition of bacterial species in a bacterial population having sialic acid, which is a biomarker, can be performed by any one of the detection methods described in the above [Detection method] section in the present embodiment.

The detection of the composition of bacterial species in the phylum *Firmicutes* (*p_Firmicutes*) in a bacterial population in a sample acquired from a subject, which is a biomarker, can be performed by any one of the detection methods described in the above [Detection method] section in the present embodiment.

In one embodiment, the data collection method for diagnosing depression in a subject may be a method that includes, before implementing any one of the detection methods described in the above [Detection method] section in the present embodiment on a sample acquired from a subject, implementing a biomarker detection method according to the above [Detection method] section in the present embodiment on a sample(s) acquired from one or more depression patients, and acquiring a biomarker reference value for depression patients.

In another embodiment, the data collection method for diagnosing depression in a subject may be a method that includes, before implementing any one of the detection methods described in the above [Detection method] section in the present embodiment on a sample acquired from a subject, implementing a biomarker detection method according to the above [Detection method] section in the present embodiment on a sample(s) acquired from one or more depression patients and a sample(s) acquired from one or more healthy individuals, and acquiring a biomarker reference value for a depression patient(s) and a biomarker reference value (normal value) for a healthy individual(s).

According to these methods, in the case in which a biomarker measurement value in a sample acquired from a subject is the same as a biomarker reference value for a depression patient(s), based on the data, the subject may be determined to be suffering from depression, or may be determined to have a high probability of suffering from depression. Alternatively, in the case in which a biomarker measurement value in a sample acquired from a subject is different from a biomarker reference value for a depression patient(s), based on the data, the subject may be determined to not be suffering from depression, or may be determined to have a high probability of not suffering from depression.

Additionally, in the case in which a biomarker measurement value in a sample acquired from a subject is different from a normal value, based on the data, the subject may be determined to be suffering from depression, or may be determined to have a high probability of suffering from depression. Alternatively, in the case in which a biomarker measurement value in a sample acquired from a subject is the same as a normal value, based on the data, the subject may be determined to not be suffering from depression, or may be determined to have a high probability of not suffering from depression.

In one embodiment, in the case in which a biomarker measurement value in a sample acquired from a subject is elevated in comparison with a normal value, based on the data, the subject may be determined to be suffering from depression or may be determined to have a high probability of suffering from depression.

In depression patients or depression patients that have been administered an antidepressant, the amount of bacteria in the class *Clostridia (c_Clostridia)* contained in a bacterial population having sialic acid in a sample is high or tends to be high in comparison with healthy individuals. Therefore, in the case in which the amount of bacteria in the class *Clostridia (c_Clostridia)* contained in a sample acquired from a subject is high, the subject can be determined to be (to have been) suffering from depression or to have a high probability of (having been) suffering from depression.

In depression patients or depression patients that have been administered an antidepressant, the amount of bacteria in the order *Clostridiales (o_Clostridiales)* contained in a bacterial population having sialic acid in a sample is high or tends to be high in comparison with healthy individuals. Therefore, in the case in which the amount of bacteria in the order *Clostridiales (o_Clostridiales)* contained in a sample acquired from a subject is high, the subject may be determined to be (to have been) suffering from depression or to have a high probability of (having been) suffering from depression.

In depression patients or depression patients that have been administered an antidepressant, the amount of bacteria in the genus *Ruminococcus* (*g_Ruminococcus*) contained in a bacterial population having sialic acid in a sample is high or tends to be high in comparison with healthy individuals. Therefore, in the case in which the amount of bacteria in the genus *Ruminococcus* (*g_Ruminococcus*) contained in a sample acquired from a subject is high, the subject may be determined to be (to have been) suffering from depression or to have a high probability of (having been) suffering from depression.

In depression patients or depression patients that have been administered an antidepressant, the amount of bacteria in the family *Erysipelotrichaceae* (*f_Erysipelotrichaceae*) contained in a bacterial population having sialic acid in a sample is low or tends to be low in comparison with healthy individuals. Therefore, in the case in which the amount of bacteria in the family *Erysipelotrichaceae* (*f_Erysipelotrichaceae*) contained in a sample acquired from a subject is low, the subject may be determined to be (to have been) suffering from depression or to have a high probability of (having been) suffering from depression.

In depression patients or depression patients that have been administered an antidepressant, the amount of bacteria in the genus *Lactobacillus* (*g_Lactobacillus*) contained in a bacterial population having sialic acid in a sample is low or tends to be low in comparison with healthy individuals. Therefore, in the case in which the amount of bacteria in the genus *Lactobacillus* (*g_Lactobacillus*) contained in a sample acquired from a subject is low, the subject may be determined to be (to have been) suffering from depression or to have a high probability of (having been) suffering from depression.

In depression patients or depression patients that have been administered an antidepressant, the amount of the bacteria *Lactobacillus reuteri* (*g_Lactobacillus s_reuteri*) contained in a bacterial population having sialic acid in a sample is low or tends to be low in comparison with healthy individuals. Therefore, in the case in which the amount of the bacteria *Lactobacillus reuteri* (*g_Lactobacillus s_reuteri*) contained in a sample acquired from a subject is low, the subject may be determined to be (to have been) suffering from depression or to have a high probability of (having been) suffering from depression.

In depression patients or depression patients that have been administered an antidepressant, the amount of bacteria in the order *Lactobacillales* (*o_Lactobacillales*) contained in a bacterial population having sialic acid in a sample is low or tends to be low in comparison with healthy individuals. Therefore, in the case in which the amount of bacteria in the order *Lactobacillales* (*o_Lactobacillales*) contained in a sample acquired from a subject is low, the subject may be determined to be (to have been) suffering from depression or to have a high probability of (having been) suffering from depression.

In depression patients or depression patients that have been administered an antidepressant, the amount of bacteria of the species *Erysipelotrichaceae* (*s_Erysipelotrichaceae*) contained in a bacterial population having sialic acid in a sample is low or tends to be low in comparison with healthy individuals. Therefore, in the case in which the amount of bacteria of the species *Erysipelotrichaceae* (*s_Erysipelotrichaceae*) contained in a sample acquired from a subject is low, the subject may be determined to be (to have been) suffering from depression or to have a high probability of (having been) suffering from depression.

In depression patients or depression patients that have been administered an antidepressant, the amount of bacteria in the phylum *Firmicutes* (*p_Firmicutes*) contained in a bacterial population in a sample is high or tends to be high in comparison with healthy individuals. Therefore, in the case in which the amount of bacteria in the phylum *Firmicutes* (*p_Firmicutes*) contained in a sample acquired from a subject is high, the subject may be determined to be (to have been) suffering from depression or to have a high probability of (having been) suffering from depression.

In depression patients or depression patients that have been administered an antidepressant, the amount of bacteria in the class *Clostridia (c_Clostridia)* contained in a bacterial population in a sample is high or tends to be high in comparison with healthy individuals. Therefore, in the case in which the amount of bacteria in the class *Clostridia (c_Clostridia)* contained in a sample acquired from a subject is high, the subject may be determined to be (to have been) suffering from depression or to have a high probability of (having been) suffering from depression.

In another embodiment, the data collection method for diagnosing depression in a subject may be a method that includes implementing any one of the detection methods described in the above [Detection method] section in the present embodiment on multiple samples acquired over time from the same subject.

According to this method, in the case in which the biomarker measurement values in the samples acquired from the subject exhibit a change at a certain time compared to before that time, based on the data, the subject may be determined to have suffered from depression, or to have a high probability of having suffered from depression. For example, through periodically implemented health checkups, etc., this method can be used to monitor depression in a subject.

In one embodiment, in the case in which the biomarker measurement values in the samples acquired from the subject increase at a certain time compared to before that time, based on the data, the subject may be determined to have suffered from depression or to have a high probability of having suffered from depression. For example, in the case in which the biomarker, which is the composition of bacterial species in a bacterial population having sialic acid, is the amount of bacteria in the class *Clostridia* (*c_Clostridia*) that is contained, this is because the amount of bacteria in the class *Clostridia* (*c_Clostridia*) contained in a bacterial population having sialic acid in a sample is high or tends to be high in depression patients in comparison with healthy individuals.

In one embodiment, depression in a subject can be diagnosed by any one of the above-mentioned determinations.

In this case, the reference value or the normal value may be the mean value or the median value of biomarker measurement values in samples acquired from subjects in the same group or acquired over time from the same subject, or may be a reference range or a normal range that has been suitably set based on biomarker measurement values.

Alternatively, the reference value or the normal value may be decided, as appropriate, by a person skilled in the art to be a value by which a subject can be determined to be suffering from depression with a desired precision based on biomarkers in samples acquired from subjects in the same group or acquired over time from the same subject.

Additionally, the biomarker measurement value in a sample acquired from a subject being the same as a reference value or a normal value, for example, means that there is no significant difference from the reference value or the normal value by a suitable statistical process. The biomarker measurement value in a sample acquired from a subject being different from a reference value or a normal value, for example, means that there is a significant difference from the reference value or the normal value by a suitable statistical process. The biomarker measurement value in a sample acquired from a subject being elevated in comparison with a reference value, for example, means that the biomarker measurement value in the sample acquired from the subject is significantly elevated in comparison with the reference value by a suitable statistical process.

The statistical processing method can be selected, as appropriate, in accordance with a method that would be well known to a person skilled in the art.

In one embodiment, the reference value may be a value that is 150%, 200%, 250%, 300%, 350%, 400%, 450%, or 500% or more with respect to the normal value, or may be decided, as appropriate, by a person skilled in the art to be a value by which a subject can be determined to be suffering from depression with a desired accuracy and/or precision.

In one embodiment, the data collection method explained above or the method for diagnosing depression based on said data collection method may be implemented in combination with another data collection method for diagnosing depression, or another method for diagnosing depression.

### [Kit]

The kit in one embodiment is a kit for diagnosing depression in a subject, the kit including means for detecting any one of the biomarkers described in the above [Biomarker] section in the present embodiment.

In one embodiment, said kit is a kit for implementing a detection method described in the above [Detection method] section in the present embodiment.

Examples and preferred examples of the "kit" are as described in "Kit" in the fourth embodiment.

According to the above-mentioned kit, the acquisition of the sample is non-invasive. Additionally, by measuring and detecting biomarkers, objective and/or quantitative test results can be acquired.

### EXAMPLES

Examples will be shown to explain the present invention in further detail below. However, the present invention should not be construed in a limiting manner due to these examples.

### [Example 1] Model animal

Model animals that were used in the examples below are as indicated below.

### (1) Chronic ultra-mild stress model mouse group

Stress-vulnerable mice (BALB/c mice) were subjected to chronic ultra-mild stress (the following stress burdens were constantly applied randomly for 24 hours: acetic acid odor, switching cages with other mice, bedding being wettened, being left in a small and confined space, having the cage tilted, crushing food to make it difficult to eat, inverting light/dark) for 6 weeks. The mice that were subjected to chronic ultra-mild stress were grouped in a chronic ultra-mild stress (CUMS) group.

### (2) Control group

Individuals of the stress-vulnerable mice (BALB/c mice) that were not subjected to the chronic ultra-mild stress in section (1) above were grouped in a control group.

### (3) Antidepressant-treated group

Mice among the chronic ultra-mild stress model mice in section (1) above that were treated by administering imipramine hydrochloride (Sigma-Aldrich) (amounts corresponding to 18 mg/kg/day were dissolved in drinking water and ingested for 3 weeks) were grouped in an antidepressant-treated (CUMS+antidepressant, IMI+CUMS) group.

### [Example 2] Analysis of bacterial composition in stool sample

Mice (n = 6) in the CUMS group, the control group, and the CUMS + antidepressant-administered group were placed in sterilized cages for 30-60 minutes, and excreted stool was sampled using sterilized tweezers. The stool was stored at -20 °C until analyzed.

From the stool, bacteria were separated by means of density gradient centrifugation. Specifically, approximately 20 mg of stool was placed in 0.5 mL of phosphate-buffered saline (PBS), then homogenized by agitating overnight at 4 °C at 750 rpm. The supernatant of the suspension was sampled and transferred to 80% w/v aqueous Nycodenz solution (Nycodenz, Serumwerk Bernburg), then centrifugated for 40 minutes at 10,000×g and at 4 °C. An intermediate layer containing bacteria was sampled and washed with PBS. The bacterial cells were eluted with 0.2 M lactose.

A sequencing library was prepared in accordance with the "16S Metagenomic Sequencing Library Preparation" protocol provided by Illumina, with the V3-V4 hypervariable region in the 16S rRNA gene as the target. The V3-V4 region was amplified by a PCR reaction using 1 µl of extracted stool microbe DNA and KAPA HiFi HotStart ReadyMix (Roche) containing 1 µM of the respective primers indicated below.
· Forward: 5'-TCGTCGCAGCGTCAGATGTGTATAAGACAGCCTACGGNGGCWGCA G-3' (SEQ ID NO: 83)
· Reverse: 5'-GTCTCGTGGGCTCGGAGATGTGTATAAGAGACAGGACTACHVGGG TATCTAATCC-3' (SEQ ID NO: 84)

The PCR product was purified using Agentcourt, AMPure XP magnetic beads (Beckman Coulter). Using 2.5 µl of the purified product, PCR was performed for a second time, using Illumina index primer, with the following reaction cycle (initial denaturation at 95 °C for 3 minutes; eight cycles of denaturation at 95 °C for 30 seconds, annealing at 55 °C for 30 seconds, and elongation at 72 °C; and final elongation at 72 °C for 5 minutes). The final product was purified using Agent AMPure XP again, and eluted with 10 mM Tris pH 8.5 (elution buffer). The purified amplicons were quantified using MultiNA (Shimadzu Corporation). Then, the amplicons were sequenced with an Illumina MiSeq 2×250 bp platform, using a MiSeq Reagent Nano Kit V2 (Illumina).

Amplicon sequence data analysis software QIIME 2 (ver. 2020/8, https://qiime2.org/, Bolyen E. et al., "Reproducible, interactive, scalable and extensible microbiome data science using QIIME 2", Nature Biotechnology 37: 852-857, 2019, doi.org/10.1038/s41587-019-0209-9) was used to perform preprocessing on the raw sequence reads. Read demultiplexing, denoise quality control, read trimming, and sequence assembly were performed by means of a DADA2 plugin. Amplicon sequence variants (ASVs) generated by the DADA2 algorithm were classified by using a q2-feature-classifier. The output artifacts were organized into phyloseq objects in R version 4.1.2 (2021-11-01), each sample was diluted at the same depth, and principal coordinate analysis (PCoA) was used to calculate the β-diversity based on the Bray-Curti dissimilarity metric.

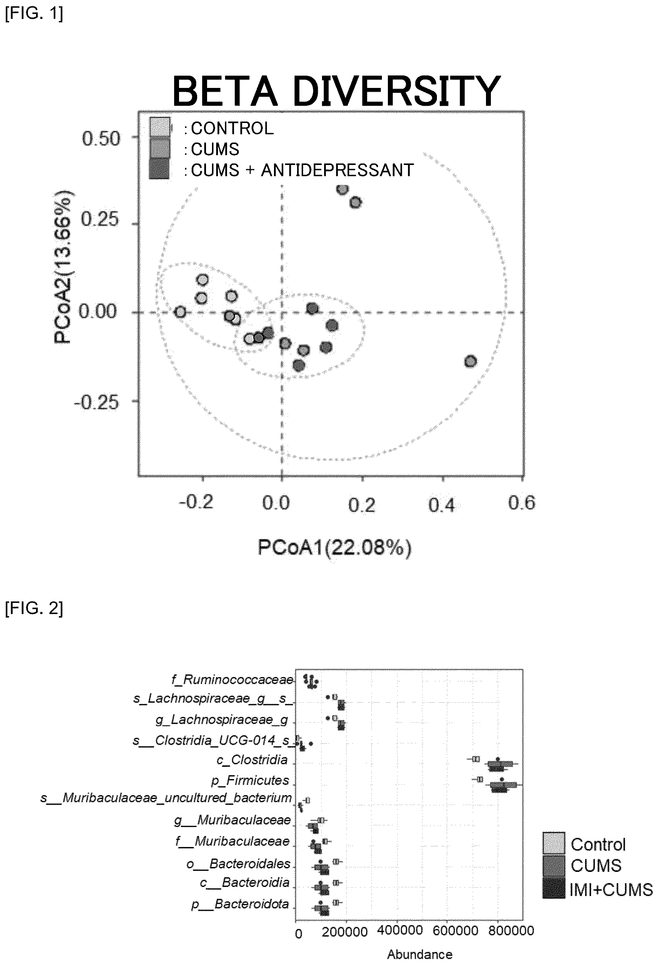

The results of the analysis (principal coordinate analysis) are shown in FIG. 1. Between the CUMS group, the control group, and the CUMS + antidepressant-administered group, the plot distributions indicating the respective individual mice were close and unseparated, and no significant difference was observed between the groups in terms of the microbiota (bacterial compositions) obtained from the stool samples.

After the PCR as described above, the sequence data obtained by sequencing with the Illumina MiSeq 2×250 bp platform using a MiSeq Reagent Nano Kit V2 (Illumina) was analyzed by LEfSe (Linear discriminant analysis Effect Size) (Segata et al., "Metagenomic biomarker discovery and explanation", Genomic Biol. 2011; 12 (6): R60, doi. 10.1186/gb-2011-12-6-r60).

As a result of analysis, it was observed that the cell count of bacteria in the phylum *Firmicutes* (*p_Firmicutes,* including the class *Clostridia* (*c_Clostridia*)) was significantly higher in the CUMS group (FIG. 2).

The composition of bacterial species in a bacterial population in a stool sample can be used as a biomarker for determining a stress level received by a subject and/or as a biomarker for diagnosing depression in a subject.

### [Example 3] Analysis of sialylated glycans in bacteria from stool by Glycan-seq

The analysis below was performed using the Glycan-seq method developed by Oinam *et al.*

Mice (n = 6) in the CUMS group, the control group, and the CUMS + antidepressant-administered group were placed in sterilized cages for 30-60 minutes, and excreted stool was sampled using sterilized tweezers. The stool was stored at -20 °C until analyzed.

From the stool, bacteria were separated by means of density gradient centrifugation. Specifically, approximately 20 mg of stool was placed in 0.5 mL of phosphate-buffered saline (PBS), then homogenized by agitating overnight at 4 °C at 750 rpm. The supernatant was sampled and transferred to 80% w/v aqueous Nycodenz solution (Nycodenz, Serumwerk Bernburg), then centrifugated for 40 minutes at 10,000×g and at 4 °C. An intermediate layer containing bacteria was sampled and washed with PBS. The bacterial cell count and size were measured using a particle counting analyzer (CDA-1000, Sysmex).

### (Preparation of DNA-barcoded lectins)

100 µg of each lectin (see the "Name" in Table 1) was dissolved in PBS (100 µl) mixed with 10 times the amount, in terms of molar ratio, of dibenzocyclooctyne-N-hydroxysuccinimidyl ester (DBCO-NHS, Funakoshi), and the result was incubated in the dark for 1 hour at 20 °C. 10 µl of 1M Tris was added to deactivate the DBCO-NHS, and the result was incubated in the dark for 15 minutes at 20 °C. Sephadex G-25 desalting columns (GE Healthcare Japan) were used to remove excess DBCO-NHS. Each DBCO-labeled lectin (100 µg/mL) was mixed with a 5'-azide-modified DNA oligonucleotide (Integrated DNA Technologies, 10 times the amount by molar ratio, SEQ ID NOs: 1-41) having a sequence corresponding to a lectin indicated in "Name" in Table 1. Furthermore, using Sepharose 4B-CL columns (Sepharose 4B-CL, GE Healthcare Japan) to which were fixed sugars for which each lectin has affinity based on glycan binding specificity, the lectins having respective glycan binding affinities were purified by affinity chromatography, and excess nucleotides were removed to prepare lectin-DNA oligonucleotide conjugates.

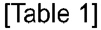

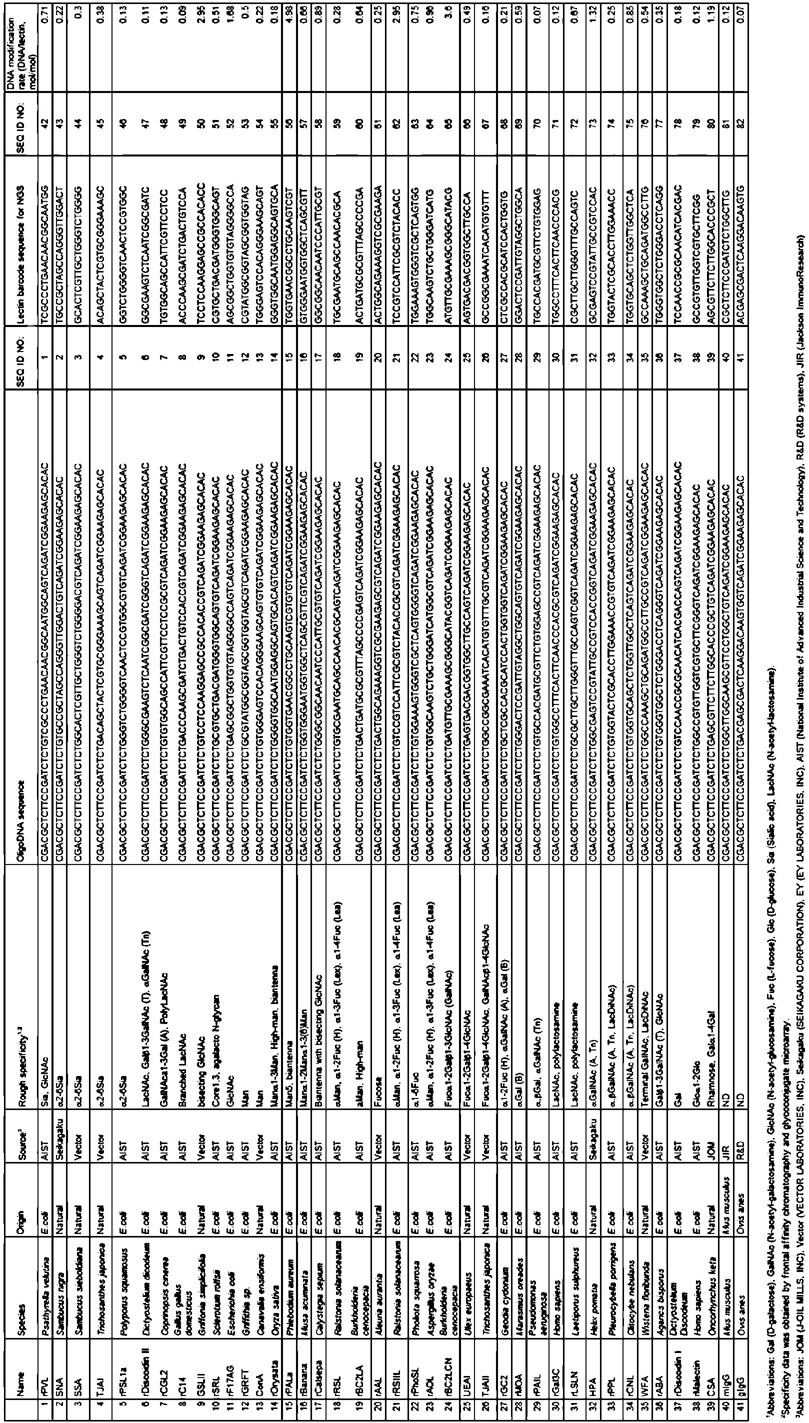

### (Glycan-seq method)

Bacterial cells (1 × 10⁷) were suspended in 1% bovine serum albumin (BSA)-containing PBS (PBS/BSA) and incubated for 1 hour at 4 °C together with two types of negative control proteins and 39 types of lectin barcoded with DNA (SEQ ID NOs: 1-41) (final concentration 0.5 µg/mL). The bacterial cells were washed three times with PBS/BSA and were irradiated for 15 minutes with UV rays, at 365 nm and 15 W, using a longwavelength blacklight UV bench lamp (UVP Blak-Ray XX-15L UV Bench Lamp, Analytik Jena). Released oligonucleotides were amplified by PCR using NEBNext Ultra II Q5 Master Mix (New England BioLabs), and an i5-index primer (Integrated DNA Technologies, Table 2 (SEQ ID NOs: 85, 87-104) and i7-index primer (Integrated DNA Technologies, Table 2 (SEQ ID NOs: 86, 105-122) including oligonucleotide sequences. The PCR was performed with 1 cycle of denaturation (45 seconds at 98 °C), 20 cycles of denaturation (10 seconds at 98 °C followed by 50 seconds at 65 °C), and 1 cycle of elongation (5 minutes at 65 °C). Due to this PCR, DNA barcodes corresponding to the various types of lectins indicated in Table 1 can be detected. Additionally, by using a primer pair with different adapters ("Bases in Adapter" in Table 2), the test groups can be differentiated for detection, even with a mixed PCR product. The PCR product was purified using an Agencourt AMPure XP Kit (Beckman Coulter). MultiNA (Shimadzu Corporation) was used to analyze the size and quality of the PCR product. The PCR product (4 nM) from each sample was treated using a MiSeq Reagent Kit v2 (Illumina), and sequenced using a MiSeq Sequencer (Illumina).

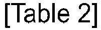

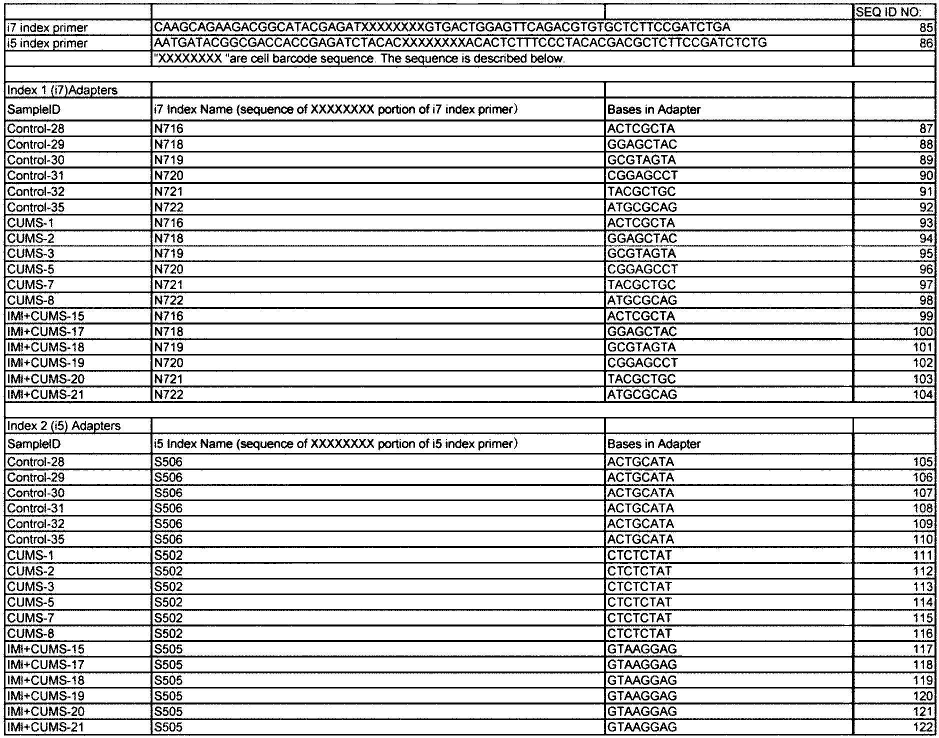

The DNA barcode information released from the lectins was directly extracted in FASTQ format.

The DNA barcode sequence corresponding to each lectin was extracted from the FASTQ format file and the DNA barcode counts were measured using a barcode DNA counting system (Mizuho Research & Technologies). The DNA barcode sequences having sequences specific to the respective lectins used to measure the DNA barcode counts are shown in "Lectin barcode sequence for NGS" (SEQ ID NOs: 42-82) in Table 1. The total number of DNA barcodes was divided by the total number of lectin barcodes and indicated as a percentage (%) for each lectin. The statistical significance levels of the lectins in Glycan-seq were evaluated by performing Holm-Bonferroni multiple hypothesis correction by means of abundance analysis of the composition data adapted from Analysis of Compositions of Microbiomes (ANCOM), and the statistically significant lectins for which p < 0.05 were selected.

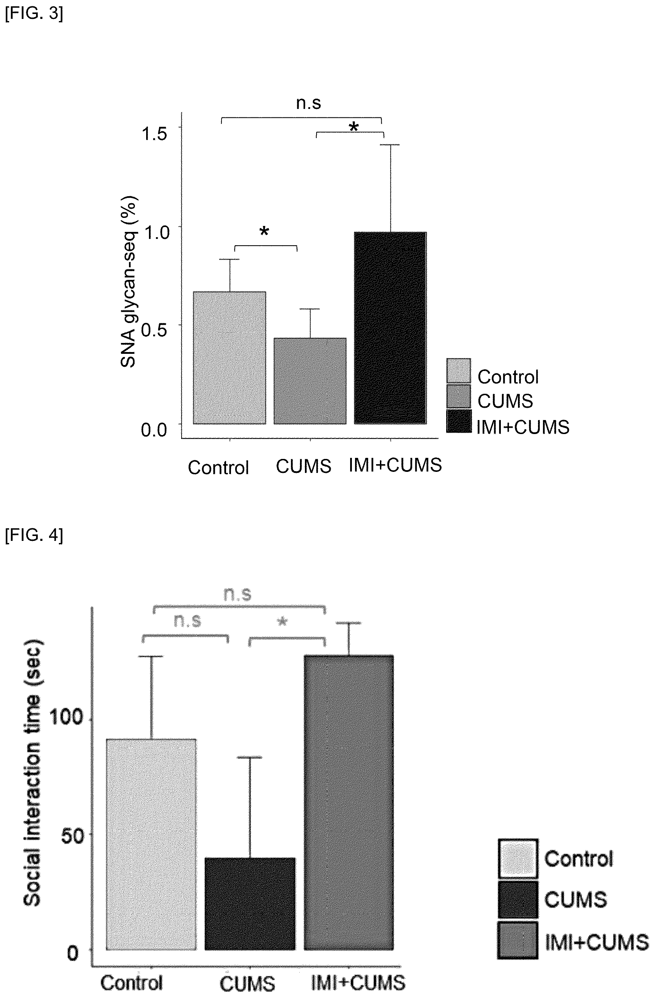

As a result thereof, the DNA barcode amount corresponding to *Sambucus nigra* lectin (elderberry) bark (SNA), which is a sialic acid-binding lectin, was significantly lower in the CUMS group in comparison with the control group and the CUMS + antidepressant-administered group. However, there was no significant difference between the CUMS + antidepressant-administered group and the control group (FIG. 3, *: p < 0.05).

This result indicates that the CUMS group had significantly lower SNA binding capacity with respect to the bacterial cells in the samples than the control group, and that, in the CUMS + antidepressant-administered group, the SNA binding capacity with respect to the bacterial cells in the samples recovered to approximately the same level as the control group.

### [Example 4] Social interaction test

A field (30 cm long, 30 cm wide, 16 cm high) having a cage (cylindrical, 10 cm in diameter, 17.5 cm high, with 23 bars and a bar spacing of 8 mm) installed at one end was prepared, and one target mouse was placed in the cage. Mice (n = 6) of each test group was placed, one at a time, in the field and the behavior was observed for 3 minutes. The shorter the time that a mouse in a test group stayed in a target area (12 cm long × 20 cm wide, centered on the target mouse) during the observation time, the more interest has declined, and this can be considered to be behavior reflecting the stress burden on the individual, or depression-like behavior.

In the CUMS group, the approach time (seconds) with respect to the target mouse during the social interaction test was significantly shorter, i.e., behavior reflecting a stress burden or depression-like behavior was strongly observed. Meanwhile, in the control group and the CUMS + antidepressant-administered group, the approach time with respect to the target mouse in the social interaction test was significantly longer. This result is shown in FIG. 4 (*: p < 0.05).

### (Lectin binding capacity and social interaction test)

The results of analysis of the correlation between the SNA binding capacity in Example 3 and the results of the social interaction test in the present example are indicated in FIG. 5.

In the CUMS group having low lectin binding capacity, the approach time with respect to the target mouse in the social interaction test was short, i.e., behavior reflecting a stress burden or depression-like behavior was strongly observed. Meanwhile, in the control group and the CUMS + antidepressant-administered group (IMI+CUMS), which had high lectin binding capacity, the approach time with respect to the target mouse in the social interaction test was long.

Thus, a significant correlation was confirmed between the SNA binding capacity and the target approach time in the social interaction test (R = 0.67, p = 0.0026).

### [Example 5] Analysis of bacterial species composition in bacterial population having sialic acid

1 µg/µl of SNA was labeled with biotin and incubated for 1 hour in a shaker set to 1,400 rpm at 4 °C together with streptavidin-bound Dynabeads (Thermo Fisher Scientific). 1 × 10⁷ bacterial cells obtained by being separated from stool from the three sample groups (control group, CUMS group, CUMS + antidepressant-administered group) (n = 6) in the same manner as in Example 3 were incubated overnight, together with washed SNA-bound beads, in a shaker set to 700 rpm at 4 °C. The bacterial cells that were bound were separated by using magnetic beads and eluted with 0.2 M lactose.

As described in Example 2, a sequencing library was prepared with the V3-V4 hypervariable region in the 16S rRNA gene as the target, and the V3-V4 region was amplified by means of a PCR reaction.

As described in Example 2, the PCR product was purified, PCR was performed for a second time, and after purification, sequencing was performed.

Furthermore, as described in Example 2, the α-diversity (Wilcoxon pairwise test, p < 0.05) and the β-diversity based on the Bray-Curti dissimilarity metric were calculated by principal coordinate analysis (PCoA).

The results of the analysis are shown in FIG. 6.

As shown in FIG. 6, the cell count of bacteria in the sialylated class *Clostridia* (*c_Clostridia*) was significantly higher in the CUMS group and in the CUMS + antidepressant-administered group (IMI+CUMS) than in the control group. Additionally, the cell counts of bacteria in the sialylated order *Clostridiales* (*o_Clostridiales*) and bacteria in the genus *Ruminococcus* (*g_Ruminococcus*) were significantly higher in the CUMS group and in the CUMS + antidepressant-administered group than in the control group.

Meanwhile, the cell counts of bacteria in the sialylated genus *Lactobacillus* (*g_Lactobacillus*)*,* bacteria in the family *Erysipelotrichaceae* (*f_ Erysipelotrichaceae*), the bacteria *Lactobacillus reuteri* (*g_Lactobacillus s_reuteri*)*,* bacteria in the order *Lactobacillales* (*o_Lactobacillales*)*,* and bacteria of the species *Erysipelotrichaceae* (*s_Erysipelotrichaceae*) were significantly higher in the control group than in the CUMS group or in the CUMS + antidepressant-administered group.

### (Open field test)

Mice of each group were placed in an open field that was 30 cm long, 30 cm wide, and 16 cm high, and the time during which the mice were in a 10-cm square field at the center was measured. This time is known to serve as an indicator of anxious behavior such that, the stronger the anxiety is, the shorter the time that is spent at the center.

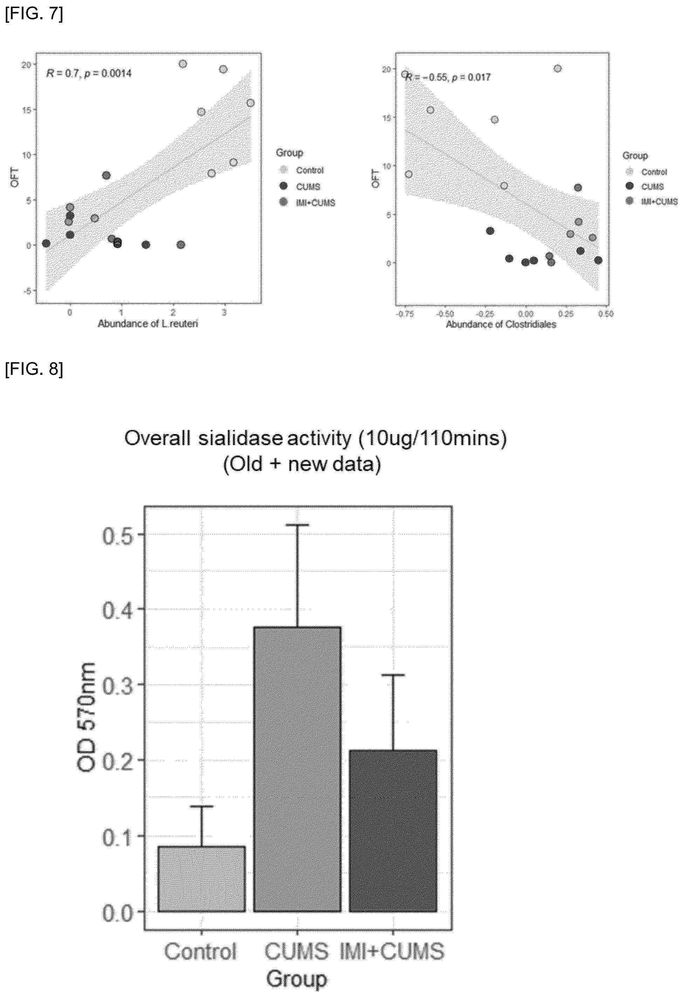

The relationships between the time (seconds, vertical axis) that the mice spent at the center in the open field test (OFT) and the cell count of bacteria of the sialylated *Lactobacillus reuteri (g_Lactobacillus s_reuteri)* and the cell count of bacteria in the sialylated order *Clostridiales* (*o_Clostridiales*) are shown in FIG. 7.

The longer the time that a mouse spent at the center in the open field test, the more the cell count of bacteria of the sialylated *Lactobacillus reuteri* (*g_Lactobacillus s_reuteri*) increased, and a significant correlation was observed (R = 0.7, p = 0.0014) (FIG. 7, left).

Additionally, the shorter the time that a mouse spent at the center in the open field test, the more the cell count of bacteria in the sialylated order *Clostridiales (o_Clostridiales)* increased, and a significant correlation was observed (R = -0.55, p = 0.017) (FIG. 7, right).

From the above results, significant differences were observed between the control group and the CUMS group and CUMS + antidepressant-administered group in terms of the cell counts of bacteria in the class *Clostridia (c_Clostridia),* the order *Clostridiales* (*o_Clostridiales*)*,* the genus *Ruminococcus* (*g_Ruminococcus*)*,* the genus *Lactobacillus* (*g_Lactobacillus*)*,* the family *Erysipelotrichaceae* (*f_Erysipelotrichaceae*)*, Lactobacillus reuteri* (*g_Lactobacillus s_reuteri*)*,* the order *Lactobacillales* (*o_Lactobacillales*)*,* and the species *Erysipelotrichaceae* (*s_Erysipelotrichaceae*) having sialic acid. The cell counts of these bacteria can be used as biomarkers for determining stress levels and/or diagnosing depression.

Additionally, the composition of bacterial species in a bacterial population having sialic acid can be used as a biomarker for determining a stress level received by a subject and/or as a biomarker for diagnosing depression in a subject.

### [Example 6] Analysis of sialidase activity

10 mg of stool was added to 1 ml of Tris-HCl buffer (0.15 M NaCl, 1% PBSTx (0.25% Triton-X 100 (PBS+Tx), pH 6.7) and homogenized on ice using a biomasher (Nippi, product code: 320103). The homogenized stool was incubated for 1 hour on ice, then centrifugated for 3 minutes at 4000 g. The supernatant was collected and passed through a Durapore membrane filter (MilliporeSigma, catalog no. UFC30GV0S), then centrifugated for 10 minutes at 15,000 rpm, and the supernatant was collected. The separated proteins were quantified using a Pierce (registered trademark) BCA Protein Assay Kit (Thermo Fisher Scientific, catalog no. 23225). For a sample containing 10 µg of proteins, the sialidase activity was measured using a Neuraminidase assay kit (Sigma-Aldrich, MAK121) in accordance with the protocol. 80 µl of the reaction solution, the sample, and 20 µl of a reference standard were added to each well in a 96-well plate (Proteosave SS 96F plate, SUMILON, catalog no. MS-8296F), and incubated, screened from light, for 80 minutes at 37 °C. After 80 minutes, a SpectraMax multimode microplate reader (Molecular Devices Japan) was used to measure the absorbance at 570 nm (colorimetric measurement).

The results of the analysis are shown in FIG. 8.

The sialidase activity was significantly higher (p < 0.05) in the CUMS group than in the control group or in the CUMS + antidepressant-administered group (IMI+CUMS).

### (Forced swim test (FST))

A cylindrical container with a height of 20 cm and a diameter of 14 cm was filled with water at 23 ± 0.5 °C to a depth of 13 cm. Mice were made to swim therein, observed for 5 minutes, and the immobility times were measured. Immobility time is an indicator of the stress burden on the individual and depression-like behavior such that, the shorter the immobility time is, the stronger the anti-stress effects and the anti-depression effects are considered to be.

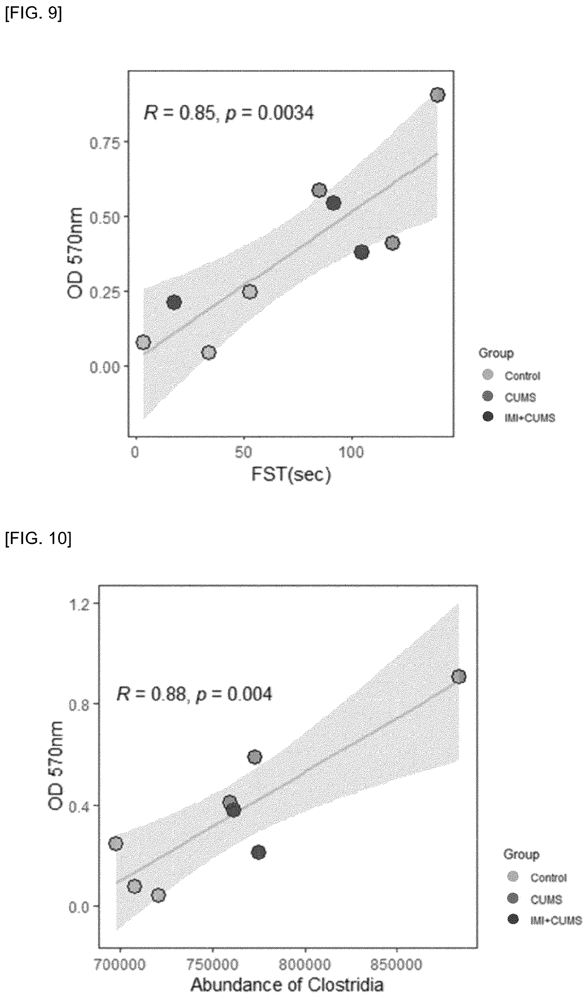

The relationship between the immobility time (seconds, horizontal axis) in the forced swim test and the sialidase activity (vertical axis) in stool samples of mice is shown in FIG. 9.

The longer the immobility time in the forced swim test was, the higher the sialidase activity was, and a significant correlation was observed (R = 0.85, p = 0.0034).

### (Cell count of bacteria in class Clostridia (c_Clostridia) and sialidase activity)

The results of analysis of the correlation between the cell count of bacteria in the class *Clostridia* (*Clostridia*) in Example 2 and the sialidase activity in the present example are shown in FIG. 10.

A significant correlation was observed between the cell count of bacteria in the class *Clostridia (c_Clostridia)* and the sialidase activity (R = 0.88, p = 0.004).

### (Cell count of bacteria in phylum Firmicutes (p_Firmicutes) and sialidase activity)

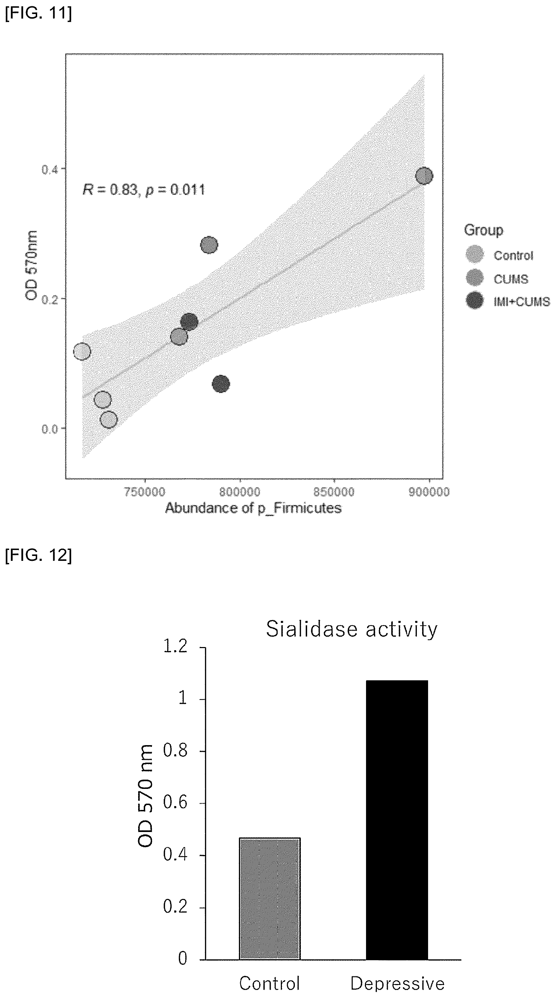

The results of analysis of the correlation between the cell count of bacteria in the phylum *Firmicutes* (*p_Firmicutes,* including the class *Clostridia* (*c_Clostridia*)) in Example 2 and the sialidase activity in the present example are shown in FIG. 11.

A significant correlation was observed between the cell count of bacteria in the phylum *Firmicutes* (*p_Firmicutes*) and sialidase activity (R = 0.83, p = 0.011).

### [Example 7] Analysis in human clinical samples

Stool samples from one healthy human (Lot No. 22-08-527) and from one depression patient (Lot No. 22-08-535) were purchased from Medix Biochemica. The conditions of the healthy person and the depression patient are as shown in Table 3.

The sialidase activity was measured in a manner similar to the measurements of mouse stool in Example 6, aside from having used test samples containing 100 µg, rather than 10 µg, of proteins as the test samples for measuring the sialidase activity.

**[Table 3]**

| Sample | Lot No. | OD 570 nm | Age | Sex | Ethnicity | Medication History |
|---|---|---|---|---|---|---|
| Healthy person | 22-08-527 | 0.4685 | 24 | male | Caucasian | - |
| Depression patient | 22-08-535 | 1.072 | 30 | female | Caucasian | - |

The results of the analysis are shown in FIG. 12 and Table 3.

The absorbance (OD) indicating sialidase activity was 0.4685 in the healthy individual (control group), whereas the absorbance (OD) indicating sialidase activity was 1.072 in the depression patient.

Thus, the sialidase activity was shown to be higher in a stool sample from a human depression patient than that from a healthy human.

From the above results, the sialidase activity from bacteria in a stool sample can be used as a biomarker for determining a stress level received by a subject and/or as a biomarker for diagnosing depression in a subject.

Additionally, in an individual that has received treatment by an antidepressant, the sialidase activity is approximately the same as that in healthy individuals. Therefore, the sialidase activity can be used as a biomarker for evaluating the efficacy of an antidepressant.

### INDUSTRIAL APPLICABILITY

The sialidase activity in a sample acquired from a subject reflects the stress level received by the subject, depression in the subject, and the efficacy of an antidepressant in the subject. Additionally, the composition of bacterial species in a bacterial population having sialic acid in a sample acquired from a subject reflects the stress level received by the subject and depression in the subject.

Therefore, the sialidase activity and the composition of bacterial species in a bacterial population having sialic acid in a sample acquired from a subject are suitable for use as markers for evaluating the stress level received by the subject, depression in the subject, and/or the efficacy of an antidepressant in the subject, thus having industrial applicability.

## Claims

1. A biomarker for determining a stress level received by a subject, wherein the biomarker is sialidase activity from bacteria in a sample acquired from the subject.

2. The biomarker according to claim 1, wherein the sample is stool.

3. The biomarker according to claim 1, wherein the bacteria comprise bacteria in class *Clostridia.*

4. A biomarker detection method, wherein the method comprises detecting the biomarker according to claim 1 in the sample acquired from the subject.

5. A data collection method for determining a stress level received by a subject, wherein the method comprises the detection method according to claim 4.

6. A kit for determining a stress level received by a subject, wherein the kit comprises means for detecting the biomarker according to claim 1.

7. A biomarker for evaluating efficacy of an antidepressant in a subject, wherein the biomarker is sialidase activity from bacteria in a sample acquired from the subject.

8. The biomarker according to claim 7, wherein the sample is stool.

9. The biomarker according to claim 7, wherein the bacteria comprise bacteria in class *Clostridia.*

10. A biomarker detection method, wherein the method comprises detecting the biomarker according to claim 7 in the sample acquired from the subject.

11. A data collection method for evaluating efficacy of an antidepressant in a subject, wherein the method comprises the detection method according to claim 10.

12. A kit for evaluating efficacy of an antidepressant in a subject, wherein the kit comprises means for detecting the biomarker according to claim 7.

13. A biomarker for determining a stress level received by a subject, wherein the biomarker is a composition of a bacterial species in a bacterial population having sialic acid in a sample acquired from the subject.

14. The biomarker according to claim 13, wherein the composition of the bacterial species in the bacterial population having sialic acid is an amount of bacteria in class *Clostridia* contained in the bacterial population having sialic acid.

15. The biomarker according to claim 13, wherein the sample is stool.

16. A biomarker detection method, wherein the method comprises detecting the biomarker according to claim 13 in the sample acquired from the subject.

17. A data collection method for determining a stress level received by a subject, wherein the method comprises the detection method according to claim 16.

18. A kit for determining a stress level received by a subject, wherein the kit comprises means for detecting the biomarker according to claim 13.
